# EUROPEAN PATENT SPECIFICATION

(11) **EP 2 393 545 B1**
(45) Date of publication and mention of the grant of the patent: **20.07.2016**
(21) Application number: 10739084.1
(22) Date of filing: 03.02.2010
(51) Int. Cl.: A61M 29/00, A61F 2/01

(54) **PERCUTANEOUS RETRIEVABLE VASCULAR FILTER**
PERKUTANER ENTFERNBARER VENENFILTER
FILTRE VASCULAIRE À EXTRACTION PERCUTANÉE

(30) Priority: 20.05.2009 US 180041 P; 03.02.2009 US 149482 P
(43) Date of publication of application: 14.12.2011
(73) Proprietor: Merit Medical Systems, Inc., South Jordan, UT 84095 (US)
(72) Inventor: HALLISEY, Michael, J., Wethersfield, CT 06109 (US)
(74) Representative: Bradley, Adrian
(86) International application number: PCT/US2010/023100
(87) International publication number: WO 2010/091118

(56) References cited:
- EP-A2- 1 987 800
- WO-A1-00/51500
- WO-A1-2009/032834
- WO-A1-2009/032834
- WO-A2-02/071977
- US-A1- 2005 015 111
- US-A1- 2005 222 604
- US-A1- 2005 288 704
- US-A1- 2006 106 417

## Description

### Field of the Invention

The present invention relates to filters within a vessel. In particular, the present invention relates to vena cava filters which may be permanent or retrievable.

### Background of the Invention

Pulmonary embolism (PE) is a common health problem and a leading cause of death in all age groups. Most pulmonary emboli result from deep vein thrombosis (DVT) in the lower extremities or pelvis. The blood clots that form in another part of the body can migrate through the veins back to the heart and into the lungs, leading to a pulmonary infarct by depriving the blood and oxygen supply to a portion of the lung. An important risk factor for the development of DVT is venostasis; common scenarios include bedridden trauma patients and passengers on long airplane flights. Other causes of DVT are hypercoagulability and vessel wall inflammation. Corriere M, et al. Vena cava filters: an update. Future Cardiol. 2(6): 695-707 (2006).

Untreated PE is associated with a high mortality rate, generally held to be about 30%, with 11% of patients dying within the first hour. Patients with recurrent PE are at much higher risk. However, when the condition is promptly treated, the survival rate increases significantly. Pulmonary embolism [on-line]. Retrieved on July 11, 2008 from http://www.mayoclinic.com/health/pulmonary-embolism/DS00429/DSECTION=complications. Anticoagulant therapy, such as heparin and warfarin, is the first line of treatment for PE. For patients in whom anticoagulation is contraindicated or inadequate, such as trauma and cancer patients, vena cava filters, including inferior vena cava (IVC) filters, provide alternative protection from PE. Corriere M, et al. Vena cava filters: an update. Future Cardiol. 2(6): 695-707 (2006). Vena cava filters are typically metal devices deployed under fluoroscopic guidance into the vena cava to prevent blood clots from migrating to the lungs. An IVC filter is usually placed below the level of the renal veins with the tip above the outflow of the renal veins. When the blood clot is captured in the top of the filter, it is then washed and lysed by the influx of the blood flow.

While some vena cava filters are permanently placed in the patient, there are potential complications associated with long-term filter implantation, including thrombotic occlusion of the vena cava, filter migration, filter fragmentation and filter embolization. Mohan C, et al. Comparative efficacy and complications of vena caval filters. J. Vase. Surge. 21:235 - 246 (1995). See also U.S. Patent No. 7,261,731. Nonpermanent filters, including temporary and retrievable filters, are recommended for patients having a limited period of risk for PE or the contraindication to anticoagulation. These types of filters are also recommended in adolescent and young-adult patients with normal life expectancy. Linsenmaier U et al. Indications, management, and complications of temporary inferior vena cava filters. Cardiovasc. Intervent. Radiol. 21(6): 464-469 (1998). Some temporary vena cava filters are attached to a wire or catheter, which is either exteriorized or secured subcutaneously for filter removal. The peripheral tether causes a certain degree of patient immobility and increases the risk of infection. Murray A, et al. Radiology 225:835-844 (2002).

In U.S. Patent No. 6,391,045, a vena cava filter is disclosed that comprises a set of helical filter-wires joined at a central region and terminating in free ends constructed to engage the vessel wall. A major mid-portion of the length of the free-ended wires are generally helical forming shape. Anchoring is accomplished by a separate assembly formed of struts and anchoring devices. A trapezoid supporting strut assembly and other means for providing linear engagement with the wall of the vena cava are also disclosed. U.S. Patent No. 6,059,825 discloses a retrievable vena cava filter formed of a single high-memory wire. The wire has a coiled cylindrical portion and a coiled conical portion. The coils of the cylindrical portion have a sufficiently large diameter contact the walls of the inferior vena cava with sufficient force to hold the coils in place against the inferior vena cava. The conical portion of the wire has a segment that aids in the removing of the filter from the vena. The vena cava filter of U.S. Patent No. 5,954,741 features an inflatable balloon at or near the distal end of an elongate flexible multiple-lumen core or stem. The balloon is deflated prior to insertion; it is inflated to become a filter when properly positioned in the vein, and finally it is deflated for removal purposes.

In the U.S.A., there are currently six FDA-approved permanent vena cava filters with different shapes, configurations, sizes and materials. They include the stainless steel Greenfield filter (Boston Scientific, Natick, MA), the Bird's Nest filter (Cook, Bloomington, IN), the Simon Nitinol Filter (Bard, Tempe, AZ), the TrapEase filter (Cordis, Miami Lakes, FL), the Vena-Tech filter (B. Braun Medical, Evanston, IL) and the G2 filter (Bard, Tempe, AZ). There are only two FDA-approved retrievable vena cava filters: the Günter-Tulip filter (Cook, Bloomington, IN) and the OptEase filter (Cordis, Miami Lakes, FL). Corriere M, et al. Vena cava filters: an update. Future Cardiol. 2(6): 695-707 (2006).

Retrievable vena cava filters are designed with specific features, so depending on the individual situation, they may either be left in the vessel permanently or be retrieved. While the versatility of retrievable filters makes them favorable options, in clinical practice, a large number of the retrievable filters are prone to migration and tilt. Filters have been reported to migrate to the heart, pulmonary vasculature, and distally, along with subsequent vascular perforation due to filter strut extrusion. Cunliffe C, et al. A fatal complication of a vena cava filter associated with pulmonary thromboembolism. Am. J. Forensic. Med. Pathol. 29: 173-176 (2008). Filter tilt seriously reduces the filtering efficiency. The tilt of greater than 14 degrees from the longitudinal axis is considered to be associated with the increased incidence of recurrent PE. Joels C, et al. Complications of inferior vena cava filters. Am Surg. 69:654-659 (2003). The migration or tilt further makes it difficult or impossible to retrieve the filter.

The document WO 02/071977A2 discloses a filter implant having a mesh which is supported by a number of struts. Further struts are provided. They are intended to be placed on an opposite side of a body aperture to the mesh in order to anchor the filter.

It is, therefore, desired to develop a retrievable vena cava filter that has high filtering capacity with no impedance to flow, is securely fixed on the vena cava wall (non-migrating and non-tilting), and can be easily retrieved. It is also advantageous to develop a retrievable filter than can be deployed at the patient's bedside without the need of fluoroscopy.

### Summary of the Invention

It is an object of the present invention to provide a filter as disclosed in the appended claims comprising a first tube having a plurality of a first set of slots, a plurality of a second set of slots, a plurality a third set of slots and a plurality of a fourth set of slots, a second tube having a plurality of a first set of expandable legs, a plurality of a second set of expandable legs, a plurality of a third set of expandable legs, and a plurality of a fourth set of expandable legs. Each leg of the first set and the second set has an end secured to the second tube and a free end. The free end of each leg in the first set is oriented in a direction opposite to the free end of each leg in the second set. Each leg of the third set and the fourth set comprises an expandable segment and has both ends secured to the second tube. Each slot of the first set on the first tube is positioned at a radial position allowing for deployment of the first set of expendable legs. Each slot of the second set on the first tube is positioned at a radial position for the deployment of the second set of expandable legs. Each slot of the third set has a radial position for the deployment of the third set of expandable legs and each slot of the fourth set has a radial position for the deployment of the fourth set of expandable legs. Each slot on the first tube is oriented parallel to the cylindrical axis of the first tube. The second tube's external diameter is less than the first tube's internal diameter. The second tube is inserted into the first tube. Each expandable leg of the first and second sets may be deployed, and a cage may be formed comprising expandable legs from the first and second sets. The cage may form a sphere shape when the expandable legs of the first and second set are deployed. Each expandable leg of the first set and the second set has at least one barb on the free end. When the filter is deployed, the barbs on the end of free legs may be inserted into a vessel wall. The expandable segment of each expandable leg in the third and the fourth sets may each form a curvilinear shape when deployed. A segment of each expandable leg of the third set and the fourth set may be attached or secured to a pin or a tube which lies within the second tube. At least one end of the first tube has at least one notch. Each of the expandable legs of the first and second sets may comprise memory metal. The expandable segments of the expandable legs of the third and the fourth sets may comprise memory metal.

The number of expandable legs in each set may range from about 2 to about 20, from about 4 to about 15, from about 4 to about 10, from about 5 to about 10, about 4, about 5, or about 6. In one embodiment, the number of expandable legs in the first set is four, A, B, C, and D, the number of legs in the second set is four, E, F, G, and H, the number of legs in the third set is four, I, J, K, and L, and the number of legs in the fourth set is four, M, N, O and P. The expandable legs in the first set are secured at radial positions along the second tube's circumference ranging from about 0° to about 90° for A, about 90° to about 180° for B, about 180° to about 270° for C, about 270° to about 360° for D. The radial positions of the first set of expandable legs, A, B, C and D may be symmetrical. The expandable legs in the second set are secured at radial positions along the second tube's circumference of about 0° to about 90° for E, about 90° to about 180° for F, about 180° to about 270° for G and about 270° to about 360° for H. The radial positions of the second set of expandable legs, E, F, G and H may be symmetrical. The radial positions of the second set of expandable legs do not correspond to the radial positions of the first set of expandable legs. The expandable legs in the third set are secured at radial positions along the second tube's circumference ranging from about 0° to about 90° for I, about 90° to about 180° for J, about 180° to about 270° for K, about 270° to about 360° for L. The radial positions of the third set of expandable legs, I, J, K and L may be symmetrical. The radial positions of the third set of expandable legs may be off-set from the radial positions of the first set of expandable legs. The expandable legs in the fourth set are secured at radial positions along the second tube's circumference ranging from about 0° to about 90° for M, about 90° to about 180° for N, about 180° to about 270° for O and about 270° to about 360° for P. The radial positions of the fourth set of expandable legs, M, N, O and P may be symmetrical. The radial positions of the fourth set of expandable legs may be off-set from the radial position of the second set of expandable legs.

The present invention further discloses, as an example not forming part of the invention, a method for retrieving the filter comprising inserting a catheter into a vessel where the filter is positioned on the vessel wall, pushing a snare through the catheter until the snare grabs the notch, pulling back on the snare to exert tension on the filter, pushing the catheter over the snare and each expandable leg of the first set until each expandable leg retracts from the vessel wall, each expandable leg of the third and the fourth set straightens and each expandable leg of the second set retracts from vessel wall, encompassing the expandable legs of the first, second, third and fourth sets in the catheter and withdrawing the filter. In one embodiment, there is one notch at each end of the tube for retrieval of the filter from either end. The filter may alternatively be retrieved from the other end using a similar mechanism.

The present invention further provides a filter comprising a first tube having a plurality of a first set, a second set and a third set of slots, a second tube having a plurality of a first set, a second set and a third set of expandable legs. Each leg of the first set and the second set has an end secured to the second tube and a free end. The free end of each leg in the first set is oriented in a direction opposite to the free end of each leg in the second set. Each leg of the third set comprises an expandable segment and has both ends secured to the second tube. The first set of slots on the first tube are positioned at a radial position allowing for deployment of the first set of expandable legs, the second set of slots on the first tube are positioned for the deployment of the second set of expendable legs, and the third set of slots on the first tube are for the deployment of the expandable segment in each leg of the third set. Each slot on the first tube is oriented parallel to the cylindrical axis of the first tube. The second tube's external diameter is less than the first tube's internal diameter. The filter is formed by inserting the second tube into the first tube. At least one end of the first tube has at least one notch for retrieval of the filter.

The present invention also discloses, as an example not forming part of the invention, a method for retrieving the filter comprising inserting a catheter into a vessel where the filter is positioned on the vessel wall, pushing a snare through the catheter until the snare grabs the notch, pulling back on the snare to exert tension on the filter, pushing the catheter over the snare and each expandable leg of the first set until each expandable leg retracts from the vessel wall, each expandable leg of the third set straightens and each expandable leg of the second set retracts from vessel wall, encompassing the expandable legs of the first, second, third and fourth sets in the catheter, and withdrawing the filter. The filter may alternatively be retrieved from the other end using a similar mechanism.

The present invention further discloses, as an example not forming part of the invention, a filter comprising a first tube having a plurality of a first set of slots and a plurality of a second set of slots, a second tube having a plurality of a first set of expandable legs and a plurality of a second set of expandable legs. Each leg of the first set and the second set has an end secured to the second tube and a free end. The free end of each leg in the first set is oriented in a direction opposite to the free end of each leg in the second set. The first set of slots on the first tube are positioned at radial positions allowing for deployment of expandable legs of the first set. The second set of slots on the first tube are positioned at radial positions allowing for deployment of expandable legs of the second set. The slots are oriented parallel to the cylindrical axis of the first tube. The second tube's external diameter is less than the first tube's internal diameter. The filter is formed by inserting the second tube into the first tube. At least one end of the first tube has at least one notch for retrieval of the filter.

The present invention also discloses, as an example not forming part of the invention, a method for retrieving the filter comprising inserting a catheter into a vessel where the filter is positioned on the vessel wall, pushing a snare through the catheter and the inner space of the second tube until the snare grabs the notch on the first tube proximal to the second set of expandable legs, pulling back on the snare to exert tension on the filter, pushing the catheter over each expandable leg of the first set until each expandable leg retracts from the vessel wall, pulling back on the snare to exert tension on the second set of expandable legs until each expandable leg of the second set retracts from vessel wall, encompassing the expandable legs of the first and second sets in the catheter, and withdrawing the filter. The filter may alternatively be retrieved from the other end using a similar mechanism.

The present invention further discloses, as an example not forming part of the invention, a filter comprising a first tube having a plurality of a first set of slots and a plurality of a first set of expandable legs, a second tube having a plurality of a second set of slots and a plurality of a second set of expandable legs. Each leg of the first set has an end secured to the first tube and a free end. Each leg of the second set has an end secured to the second tube and a free end. The free end of each leg in the first set is oriented in a direction opposite to the free end of each leg in the second set. The first set of slots on the first tube are positioned at a radial position allowing for deployment of the second set of expandable legs on the second tube; the second set of slots on the second tube are positioned for the deployment of the first set of expendable legs on the first tube. Each slot is oriented parallel to the cylindrical axis of the tubes. The filter is formed by inserting the first set of expandable legs on the first tube into the second tube, and the second set of expandable legs on the second tube into the first tube, so that the first set of expandable legs on the first tube are deployed through the second set of slots on the second tube, and the second set of expandable legs on the second tube are deployed through the first set of slots on the first tube. At least one end of the first or second tube has at least one notch for retrieval of the filter. The present invention also provides a method for retrieving the filter comprising the steps of: inserting a catheter into a vessel where the filter is positioned on the vessel wall, pushing a snare through the catheter and the inner space of the filter until the snare grabs the notch on the second tube proximal to the second set of expandable legs, pulling back on the snare to exert tension on the filter, pushing the catheter over each expandable leg of the first set until each expandable leg of the first set retracts from the vessel wall, pulling back on the snare and the second tube to exert tension on the second set of expandable legs until each expandable leg of the second set retracts from vessel wall, encompassing the expandable legs of the first and second sets in the catheter, and withdrawing the filter. The filter may alternatively be retrieved from the other end using a similar mechanism.

### Brief Description of the Drawings

Figure 1a shows a perspective view of one embodiment of the filter.
Figure 1b shows a side view of the filter in Figure 1a.
Figure 1c shows a perspective view of the filter in Figure 1a as it would appear looking from 25 to 26.
Figure 2a shows a side view of the first tube in Figure 1a.
Figure 2b shows a perspective view of the first tube in Figure 2a.
Figure 2c shows a second perspective view of the first tube in Figure 2a.
Figure 2d shows a perspective view of the first tube in Figure 2a as it would appear looking from 25 to 26.
Figure 3a shows a side view of the second tube in Figure 1a.
Figure 3b shows a perspective view of the second tube in Figure 3a.
Figure 3c shows a perspective view of the second tube in Figure 3a as it would appear looking from 28 to 29.
Figure 4 illustrates one embodiment of the second tube where the third and fourth set of expandable legs are welded to a third tube or pin.
Figure 5 shows various embodiments of the barb design.
Figure 6a shows a side view of another embodiment of the filter.
Figure 6b shows a perspective view of the filter in Figure 6a.
Figure 6c shows a perspective view of the filter in Figure 6a as it would appear looking from 53 to 54.
Figure 7 shows a side view of the first tube of the filter in Figure 6a.
Figure 8a shows a side view of the second tube of the filter in Figure 6a.
Figure 8b shows a perspective view of the second tube in Figure 8a as it would appear looking from 58 to 59.
Figure 9a shows a perspective view of a filter not forming part of the invention as claimed.
Figure 9b shows a side view of the filter in Figure 9a.
Figure 9c shows a perspective view of the filter in Figure 9a as it would appear looking from 98 to 99.
Figure 10 shows a side view of the first tube of the filter in Figure 9a.
Figure 11a shows a side view of the second tube of the filter in Figure 9a.
Figure 11b shows a perspective view of the second tube in Figure 11a as it would appear looking from 101 to 102.
Figure 12a shows a perspective view of a filter not forming part of the invention as claimed.
Figure 12b shows a side view of the filter in Figure 12a.
Figure 12c shows a perspective view of the filter in Figure 12a as it would appear looking from 128 to 125.
Figure 13 shows a side view of the first tube of the filter in Figure 12a.
Figure 14 shows a side view of the second tube of the filter in Figure 12a.
Figure 15 shows deployment of the filter in Figure 1a in the inferior vena cava.
Figure 16 shows retrieval of the filter shown in Figure 1a.
Figure 17 shows the configuration of various forms of the notch.
Figure 18 shows retrieval of the filter shown in Figure 6a.
Figure 19 shows retrieval of the filter shown in Figure 9a.

### Detailed Description of the Invention

The present invention provides a vena cava filter (the "filter") which may be permanent or retrievable and which may be used for the temporary or permanent prevention of pulmonary embolism (PE). The filter can be inserted into the body percutaneously through a vein such as the femoral vein. The filter has a tube-within-tube structure that can yield semispheres which may or may not overlap upon deployment. Together the semispheres form a cage upon deployment. The filter is positioned within the vena cava at or below the juncture of the renal vein. The semi-spheres or cages of the filter ensure stable and non-migrating vena cava filtration. Because the semi-spheres are collapsible, the filter can easily be retrieved from the vena cava. Moreover, the design of the present filter makes it possible to retrieve the filter from either end.

The filter is formed from a first tube and a second tube. The first tube has a plurality of a first set of slots, a plurality of a second set of slots, a plurality of a third set of slots and a plurality of a fourth set of slots. The second tube has a plurality of a first set of expandable legs, a plurality of a second set of expandable legs, a plurality of a third set of expandable legs, and a plurality of a fourth set of expandable legs. Each leg of the first set and the second set has an end secured to the second tube and a free end. The free end of each leg in the first set is oriented in a direction opposite to the free end of each leg in the second set. Each leg of the third set and the fourth set comprises an expandable segment and has both ends secured to the second tube. A segment of each expandable leg of the third set and the fourth set may be attached or secured to a pin or a tube which lies within the second tube. The radial position of one set of expandable legs may be off-set from the radial position of a different set of expandable legs or may be the same. For example, the radial position of the third set of expandable legs may be off-set from the radial position of the first set of expandable legs or may be the same. The radial position of the fourth set of expandable legs may be off-set from the radial position of the second set of expandable legs or may be the same. Each slot of the first set of slots on the first tube is positioned at a radial position allowing for deployment of each expandable leg of the first set. Each slot of the second set of slots on the first tube is positioned at a radial position allowing for deployment of each expandable leg of the second set. Each slot of the third set of slots on the first tube being positioned at a radial position allowing for deployment of the expandable segment in each leg of the third set of expandable legs. Each slot of the fourth set of slots on the first tube being positioned at a radial position allowing for deployment of the expandable segment in each leg of the fourth set of expandable legs. Each slot on the first tube is oriented parallel to the cylindrical axis of the first tube. The second tube's external diameter is less than the first tube's internal diameter allowing the second tube to be inserted into the first tube. The diameter of the first tube may be the same as the diameter of the second tube or may be different.

In one embodiment, the number of expandable legs in the first set is four, A, B, C, and D, the number of legs in the second set is four, E, F, G, and H, the number of legs in the third set is four, I, J, K, and L and the number of legs in the fourth set is four, M, N, O and P. The expandable legs in the first set are secured at radial positions along the second tube's circumference ranging from about 0° to about 90° for A, about 90° to about 180° for B, about 180° to about 270° for C, about 270° to about 360° for D. In one embodiment, the radial positions of the first set of expandable legs are symmetrical, e.g., A is at 0°, B is at 90°, C is at 180° and D is at 270°. The radial positions of the first set of expandable legs may also be asymmetrical.

In a further embodiment, the expandable legs in the third set are secured at radial positions along the second tube's circumference ranging from about 0° to about 90° for I, about 90° to about 180° for J, about 180° to about 270° for K, about 270° to about 360° for L. The radial positions of the third set of expandable legs, I, J, K and L may be symmetrical or asymmetrical. The radial positions of the third set of expandable legs may be off-set from the radial position of the first set of expandable legs. For example, if the radial positions of the first set of expandable legs are 0° for A, 90° for B, 180° for C and 270° for D, and the third set of expandable legs are off-set 10° from the first set of expandable legs, then the third set of expandable legs are positioned at about 10° for I, about 100° for J, about 190° for K and about 280° for L. The radial positions of the third set of expandable legs may be the same as or may differ from the first set of expandable legs symmetrically or asymmetrically.

In a third embodiment, the legs in the second set are secured at radial positions along the second tube's circumference ranging from about 0° to about 90° for E, about 90° to about 180° for F, about 180° to about 270° for G and about 270° to about 360° for H. The radial positions of the second set of expandable legs, E, F, G and H may be symmetrical or asymmetrical. The radial positions of the second set of expandable legs, E, F, G and H may be the same as or may differ from the radial positions of the first set of expandable legs, A, B, C and D.

In a fourth embodiment, the expandable legs in the fourth set are secured at radial positions along the second tube's circumference ranging from about 0° to about 90° for M, about 90° to about 180° for N, about 180° to about 270° for O and about 270° to about 360° for P. The radial positions of the fourth set of expandable legs, M, N, O and P may be symmetrical or asymmetrical. The radial positions of the fourth set of expandable legs may be off-set from the radial position of the second set of expandable legs. For example, if the radial positions of the second set of expandable legs are 0° for E, 90° for F, 180° for G and 270° for H, and the fourth set of expandable legs are off-set 10° from the first set of expandable legs, then the third set of expandable legs are positioned at about 10° for M, about 100° for N, about 190° for O and about 280° for P. The radial positions of the fourth set of expandable legs may be the same as or may differ from the second set of expandable legs symmetrically or asymmetrically.

The radial positions of the second set of expandable legs may be the same as the radial positions of the first set of expendable legs or may be off-set. The radial positions of the fourth set of expandable legs may be the same as the radial positions of the third set of expandable legs or may be off-set. In one embodiment, the radial positions of the second set of expandable legs are the same as the radial positions of the first set of expendable legs; the radial positions of the third set of expandable legs are off-set from the radial positions of the first set of expandable legs; and the radial positions of the fourth set of expandable legs are the same as the radial positions of the third set of expandable legs. The free end of each leg in the first set may be oriented in a direction opposite to the free end of each leg in the second set. Alternatively, the free end of each leg in the first set may be oriented in a direction the same as the direction of the free end of each leg in the second set.

The present invention further provides a filter comprising a first tube having a plurality of a first set of slots, a plurality of a second set of slots and a plurality of a third set of slots, and a second tube having a plurality of a first set of expandable legs, a plurality of a second set of expandable legs and a plurality of a third set of expandable legs. Each leg of the first set and the second set has an end secured to the second tube and a free end. The free end of each leg in the first set is oriented in a direction opposite to the free end of each leg in the second set. Each leg of the third set comprises an expandable segment and has both ends secured to the second tube. The first set of slots on the first tube are positioned at radial positions allowing for deployment of the first set of expandable legs. The second set of slots on the first tube are positioned at radial positions allowing for the deployment of the second set of expendable legs. The third set of slots on the first tube are positioned at radial positions allowing for the deployment of the expandable segments of the third set. Each slot on the first tube is oriented parallel to the cylindrical axis of the first tube. The radial position of one set of expandable legs may be off-set from the radial position of a different set of expandable legs or may be the same. For example, the radial positions of the second set of expandable legs may be the same as or be off-set from the radial positions of the first set of expandable legs. The radial positions of the third set of expandable legs may be the same as or be off-set from the radial positions of the first set of expandable legs. In one embodiment, the radial positions of the second set of expandable legs are the same as the radial positions of the first set of expandable legs, and the radial positions of the third set of expandable legs are off-set from the radial positions of the first set of expandable legs. The second tube's external diameter is less than the first tube's internal diameter. The filter may be formed by inserting the second tube into the first tube. The diameter of the first tube may be the same as the diameter of the second tube in an example not forming part of the invention or may be different. The free end of each leg in the first set is oriented in a direction opposite to the free end of each leg in the second set. Alternatively, in an example not forming part of the invention, the free end of each leg in the first set may be oriented in a direction the same as the direction of the free end of each leg in the second set.

The present invention also provides a filter comprising a first tube having a plurality of a first set of slots and a plurality of a second set of slots, and a second tube having a plurality of a first set of expandable legs, and a plurality of a second set of expandable legs. Each leg of the first set and the second set has an end secured to the second tube and a free end. The free end of each leg in the first set is oriented in a direction opposite to the free end of each leg in the second set. The first set of slots on the first tube are positioned at radial positions allowing for deployment of the first set of expandable legs. The second set of slots on the first tube are positioned at radial positions allowing for the deployment of the second set of expendable legs. Each slot on the first tube is oriented parallel to the cylindrical axis of the first tube. The radial positions of the second set of expandable legs may be the same as or be off-set from the radial positions of the first set of expandable legs. In one embodiment, the radial positions of the second set of expandable legs are the same as the radial positions of the first set of expandable legs. The second tube's external diameter may be less than the first tube's internal diameter. The filter may be formed by inserting the second tube into the first tube. The free end of each leg in the first set may be oriented in a direction opposite to the free end of each leg in the second set. Alternatively, the free end of each leg in the first set may be oriented in a direction the same as the direction of the free end of each leg in the second set.

A cage may be formed comprising the expandable legs of the first and second sets. The cage may form a sphere shape when the expandable legs of the first and second sets are deployed. The expandable segments of the expandable legs in the third or fourth set may form a curvilinear shape when deployed. A segment of each expandable leg of the third or fourth set may be attached or secured to a pin or a tube which lies within the second tube.

In a further embodiment of the present invention, at least one end of the first tube has at least one notch. In a preferred embodiment, there is one notch at each end of the first tube for retrieval of the filter from either end. Prior to insertion into the vena cava, the filter may be encased in a catheter. The free ends of each expandable leg may have at least one barb. The legs may have various shapes, including rectangular strips, wires, tubes, rods, threads, or any other desired structure. The legs may be straight, curved, tapered or have multiple angles. For example, the leg may be curved inward at its free end to reduce penetration into the vessel wall. The shape, configuration or dimension of various portions of each leg may vary or be the same. The shape, configuration, dimension or angle of different legs of the filter may be different or the same. The legs may be notched, barbed, hooked or in any other structure that anchors the legs in the vessel wall without interfering with the retrieval of the filter. The number of expandable legs in each set may range from 2 - 20, from 4 - 15, from 4 - 10, or from 5 - 10. The number of legs in each set may be three, four, five, six or any other number that is able to ensure the stability of the filter when deployed and efficient vena cava filtration. There may be an equal or unequal number of legs in each of the first, second, third and fourth sets. The legs may be positioned symmetrically or asymmetrically at radial positions along the circumference of the tube. If the legs are positioned symmetrically, then the radial distance between each pair of legs, e.g., A-B and B-C is equal. The radial positions listed for the legs here are only provided for illustration purposes and the legs may be positioned by one of ordinary skill in the art without undue experimentation at any point along the circumference of the tube. For example, if there are 8 legs in the first expandable set, the positioning of the legs may be determined by dividing 360° by N where N is the number of legs. Where N = 8, the legs may be positioned symmetrically at 45° intervals around the circumference of the tube. The expandable legs may then be positioned at off-set intervals on the circumference different from the 45° intervals, i.e., 0° (360°), 45°, 90°, 135°, 180°, 225°, 270°, 315°.

The present invention further provides a filter comprising a first tube having a plurality of a first set of slots and a plurality of a first set of expandable legs, and a second tube having a plurality of a second set of slots and a plurality of second set of expandable legs. Each leg of the first set has an end secured to the first tube and a free end. Each leg of the second set has an end secured to the second tube and a free end. The free end of each leg in the first set is oriented in a direction opposite to the free end of each leg in the second set. The first set of slots on the first tube are positioned at a radial position allowing for deployment of the second set of expandable legs on the second tube, the second set of slots on the second tube are positioned for the deployment of the first set of expendable legs on the first tube. Each slot is oriented parallel to the cylindrical axis of the tubes. The radial positions of the first set of slots are off-set from the radial positions of the first set of expandable legs on the first tube. The radial positions of the second set of slots are off-set from the radial positions of the second set of expandable legs on the second tube. The diameter of the first tube may be the same as the diameter of the second tube or may be different. The filter is formed by inserting the first set of expandable legs into the second tube, and the second set of expandable legs into the first tube, so that the first set of expandable legs on the first tube are deployed through the second set of slots on the second tube, and the second set of expandable legs on the second tube are deployed through the first set of slots on the first tube. After deployment, the free end of each leg in the first set is oriented in a direction opposite to the free end of each leg in the second set. At least one end of the first or second tube has at least one notch for retrieval of the filter. A cage may be formed comprising the expandable legs of the first and second sets. The cage may form a sphere shape when the expandable legs of the first and second sets are deployed. In a further embodiment of the present invention, one end of the first tube and/or one end of the second tube have at least one notch. In a preferred embodiment, there is one notch at one end of the first tube and one notch at one end of the second tube for retrieval of the filter from either end. The free end of each leg in the first set may be oriented in a direction opposite to the free end of each leg in the second set. Alternatively, the free end of each leg in the first set may be oriented in a direction the same as the direction of the free end of each leg in the second set.

In one embodiment, the filter comprises a plurality of a first set of expandable legs and a plurality of a second set of expandable legs. A plurality of legs divide the space within a vessel so as to capture clots of clinically meaningful dimensions that put a patient at risk. The free end of each leg in the first set is oriented in a direction opposite to the free end of each leg in the second set. The opposing direction of the legs help to capture clinically meaningful clots. Alternatively, the free end of each leg in the first set may be oriented in a direction the same as the direction of the free end of each leg in the second set. The radial positions of the second set of expandable legs are off-set from the radial positions of the first set of expandable legs. The off-set orientation optimizes clot capture. The number of legs in each set may be five, six or any other suitable number. The legs are connected by a central positioning bar to provide a discontinuous wall contact while optimizing clot capture. The two sets of legs optimize filtration while minimizing wall contact and maximizing clot capture. The lack of longitudinal connectors between legs of two sets provides discontinuous vessel wall contact, and reduces length of contact of the filter with the vessel wall. Either set of legs forms a curved shape, which may or may not be to the degree of sphere. A leg may be curved inward at its free end to reduce penetration into cava wall. Force load is distributed over the length of leg contacting vessel wall (i.e., greater than single point contact). The force projected by curved legs is less than the force to penetrate cava wall. Interrupted legs provide minimal wall incorporation. The shape and length of the legs help maintain "pivot-free" from the placement position (i.e., prevents relative re-positioning of filter) (self-centering filter). The width of the leg may be less than the width of the corresponding receiving slot in the outer tube. The positioning bar is a longitudinal bar which engages two sets of legs for positioning access for placement and retrieval. The positioning bar allows axial centering within the vessel, while preventing "axial tilt". The tolerance for inaccuracies in final deployment is less than acceptable clinical range (about 15° tolerance). The free end of the leg may have at least one barb, which serves as an anchor and minimizes tissue disruption.

The entire deployment process of the filter can be controlled. The design of the filter provides tolerance for inaccuracies in final deployment in terms of clot trapping. Repositioning contact with device may be maintained, allowing repositioning of the filter. The filter may be retrieved through a femoral vein.

In another embodiment, the filter has a tube-within-tube structure, where the inner tube slides within the outer tube. The elements of the inner tube can be manipulated by changing the relative position of the two tubes (i.e., the legs on the inner tube expand through slots in the outer tube when properly positioned). The present filter comprises a plurality of a first set of expandable legs and a plurality of a second set of expandable legs. The free end of each leg in the first set is oriented in a direction opposite to the free end of each leg in the second set. The radial positions of the second set of expandable legs are off-set from the radial positions of the first set of expandable legs. The legs are connected by a central positioning bar. There is a closing mechanism on the inner tube. The closing mechanism is movable through slots in the outer tube, and, therefore, expands through slots to open legs on the inner tube and collapses within slots to close legs on the inner tube. The closing mechanism may control closing of the distal set of legs. The closing mechanism may protrude out of a portion of the proximal set of legs, and may get in touch with the catheter prior to the proximal set of legs does. The closing mechanism can be a plurality of legs which may form a curvilinear shape when deployed (e.g., formed in the shape of an egg beater). The closing mechanism may be a component formed in the shape of an L, a bicycle handle, a triangle, or any other suitable shape that facilitates the closing of at least one set of legs during retrieval of the filter. For example, when the closing mechanism is in the shape of a triangle, the triangle can be longer on one side and shorter on the other. During retrieval, as a catheter is pushed over the filter, the catheter first gets in touch with the taller side of the triangle, and then continues to push the triangle forward and into the slots of the outer tube. The triangle is further driven down by the catheter into the outer tube, thereby closing the distal set of legs. This filter provides good clinical utility in deployment and retrieval through easy manipulation and repositioning control. During delivery, the tube may be pushed with a ratcheting system. A catheter covers both sets of legs in closed position during delivery. After filter is positioned in the vessel, the catheter is pulled back for leg opening. The catheter may have at least one radiopaque compression ring. During retrieval, the closing mechanism on the inner tube collapses within slots to close legs on the inner tube. A catheter is advanced to engage proximal closing mechanism to collapse distal legs first; the catheter is then advanced to collapse proximal legs and continues over collapsed distal legs. A ratchet control mechanism may be used to control the catheter.

In a further embodiment, the filter comprises a plurality of a first set of expandable legs and a plurality of a second set of expandable legs. The free end of each leg in the first set is oriented in a direction opposite to the free end of each leg in the second set. The radial positions of the second set of expandable legs are off-set from the radial positions of the first set of expandable legs. The legs are connected by a central positioning bar. During delivery, the tube may be pushed with a ratcheting system; the catheter encasing the filter may be pulled back for distal delivery. The filter may be retrieved with a snare and a catheter. The snare extends from end of a tube, and is off axis (i.e., snare is not centered via the tube). The snare extends past distal legs and is pulled back to collapse distal legs. The catheter advances to collapse proximal legs and then extends over distal legs.

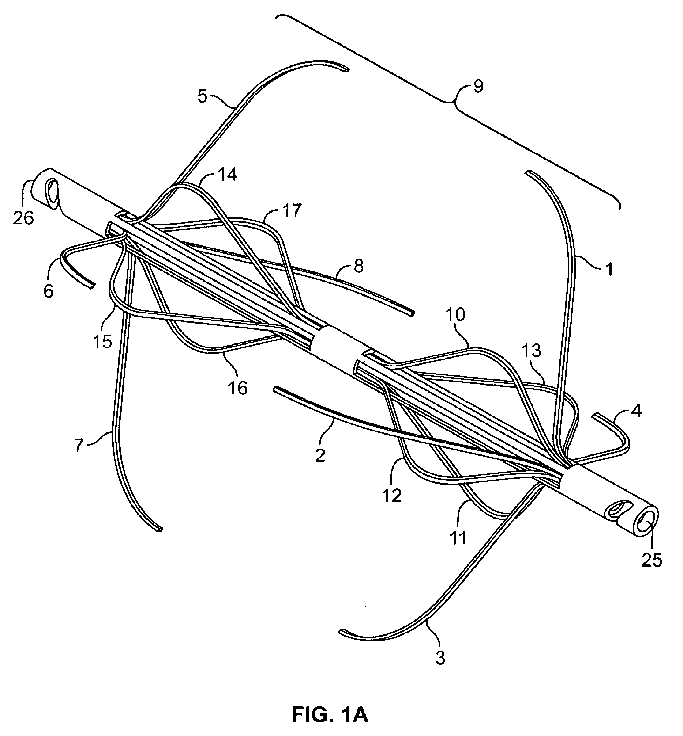

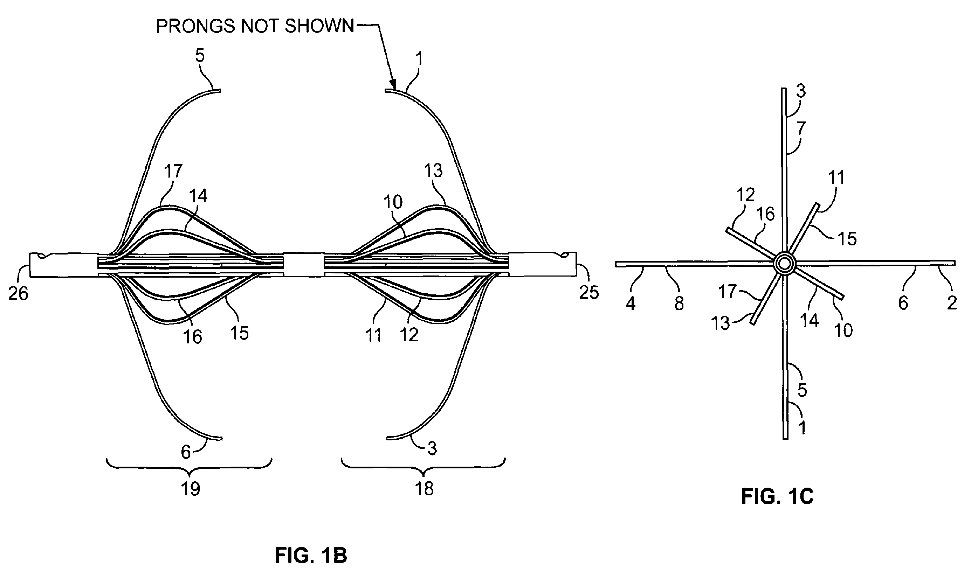

One embodiment of the assembled filter of the present invention is shown in Figures 1a, b and c. The filter comprises two tubes, a first tube and a second tube that is inserted into the first tube to form the filter. The external diameter of the second tube is smaller than the internal diameter of the first tube. The filter is formed from two sets of four expandable legs, the first expandable set, 1, 2, 3, 4 and the second expandable set, 5, 6, 7, 8. These expandable legs form a cage 9. The cage 9 may take the shape of a ball or sphere when deployed. The filter comprises a first set of expandable legs, 1, 2, 3, 4, a second set of expandable legs 5, 6, 7, 8, a third set of expandable legs 10, 11, 12, 13, and a fourth set of expandable legs 14, 15, 16, 17. The expandable segment of each leg in the third set 10, 11, 12, 13 and the expandable segment of each leg in the fourth set of legs 14, 15, 16, 17 may each form a curvilinear shape 18 and 19 when deployed (Figure 1b). As is apparent from Figure 1c, the third set of expandable legs, 10, 11, 12, 13 are positioned at different radial points along the circumference of the first tube as compared with the first set of expandable legs, 1, 2, 3 and 4. The fourth set of expandable legs, 14, 15, 16 and 17 are positioned at different radial points along the circumference of the first tube as compared with the second set of expandable legs, 5, 6, 7 and 8. In this embodiment, the second set of expandable legs, 5, 6, 7 and 8, are positioned at the same radial points as the first set of expandable legs, 1, 2, 3 and 4, while the fourth set of expandable legs, 14, 15, 16 and 17 are positioned at the same radial points as the third set of expandable legs, 10, 11, 12 and 13.

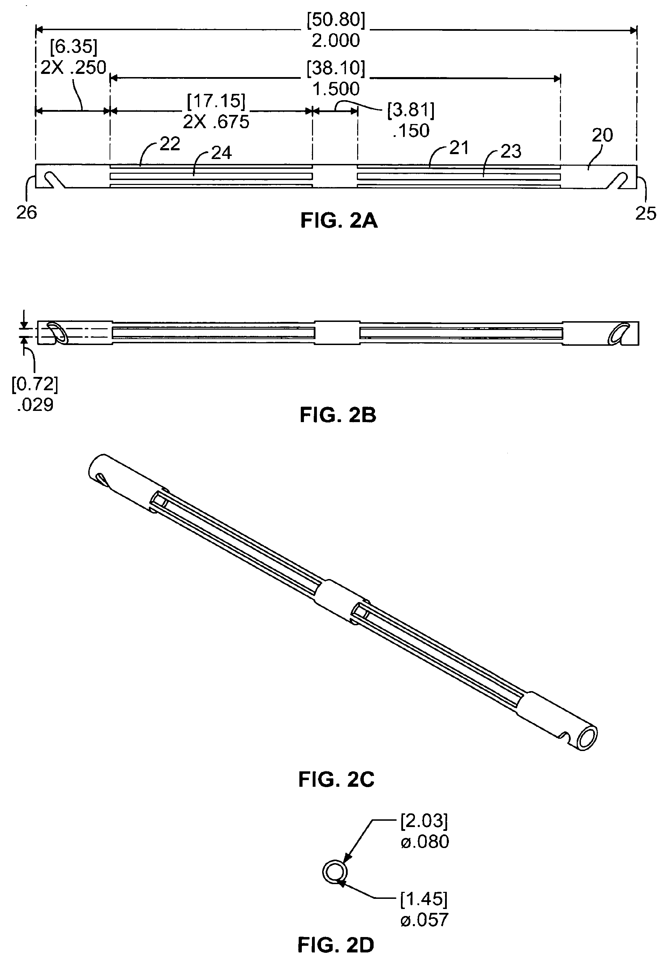

Figures 2a, b, c and d show various perspective views of the first tube. The first tube 20 contains a plurality of a first set of slots 21, a plurality of a second set of slots 22, a plurality of a third set of slots 23 and a plurality of a fourth set of slots 24. The slots are parallel to the long or cylindrical axis of the first tube. The first tube 20 has two ends, 25 and 26. The first set 21 and third set 23 of slots are positioned proximal to the end 25 of the first tube 20. The second 22 and the fourth 24 set of slots are positioned proximal to the end 26 of the first tube 20. The first set of slots may start at a position from the end 25 of the first tube from about 2 mm to about 15 mm, from about 4 mm to about 8 mm or from about 5 mm to about 7 mm or about 6 mm. Each slot of the first and second sets may range in length from about 4 mm to about 35 mm, from about 10 mm to about 25 mm, from about 15 mm to about 20 mm, or about 17 mm. The length of the first set of slots and the length of the second set of slots may be the same or may be different. The third and fourth set of slots may range in length from about 3 mm to about 30 mm, from about 5 mm to about 20 mm, from about 10 mm to about 18 mm, or about 15 mm. The length of the third set of slots and the length of the fourth set of slots may be the same or may be different.

The first tube may have a length of about 35 mm to about 80 mm, from about 40 mm to about 70 mm, from about 45 mm to about 60 mm or about 50 mm. The internal diameter of the first tube may range from about 1.0 mm to about 1.6 mm, from about 1.2 mm to about 1.6 mm, from about 1.4 mm to about 1.5 mm or about 1.45 mm. The thickness of the first tube may range from about 0.4 mm to about 0.8 mm, from about 0.5 mm to about 0.7 mm, from about 0.5 mm to about 0.6 mm, or about 0.58 mm. The thickness of the first tube may be constant or may vary from one end to the other end. Either end of the first tube may be straight or beveled.

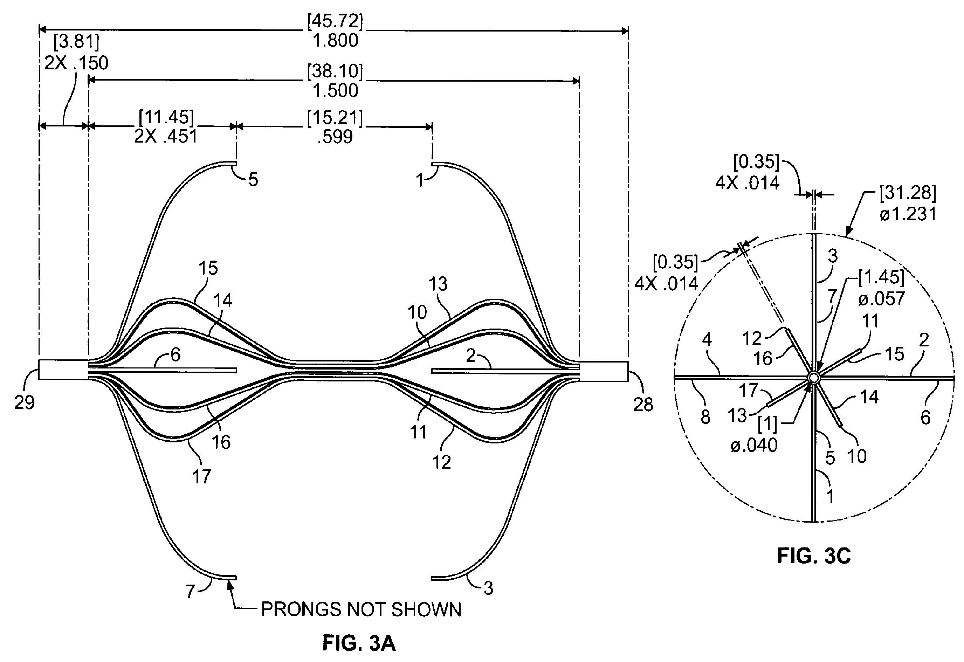

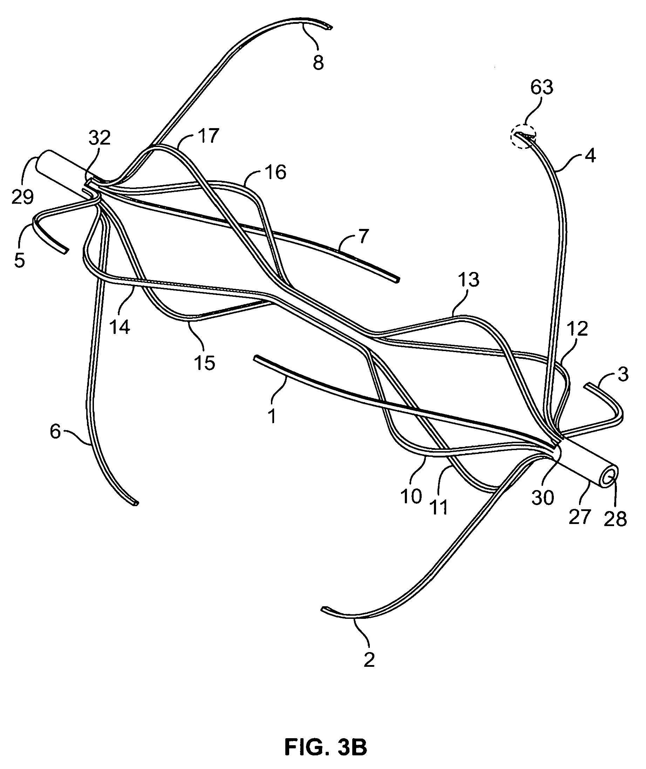

Figures 3a, b and c show various perspective views of the second tube. The second tube 27 comprises four sets of expandable legs and two ends 28, 29. The first set of expandable legs 1, 2, 3, 4, and the third set of expandable legs 10, 11, 12, 13, are secured proximal to the end 28 of the second tube 27. The second set of expandable legs 5, 6, 7, 8, and the fourth set of expandable legs 14, 15, 16, 17, are secured proximal to the end 29. As is apparent from Figure 3c, the third set of expandable legs, 10, 11, 12, 13 are positioned at different radial points along the circumference of the second tube 27 as compared with the first set of expandable legs, 1, 2, 3 and 4. The fourth set of expandable legs, 14, 15, 16 and 17 are positioned at different radial points along the circumference of the second tube 27 as compared with the second set of expandable legs, 5, 6, 7 and 8. In this embodiment, the second set of expandable legs, 5, 6, 7 and 8, are positioned at the same radial points along the circumference of the second tube 27 as the first set of expandable legs, 1, 2, 3 and 4, while the fourth set of expandable legs, 14, 15, 16 and 17 are positioned at the same radial points along the circumference of the second tube 27 as the third set of expandable legs, 10, 11, 12 and 13.

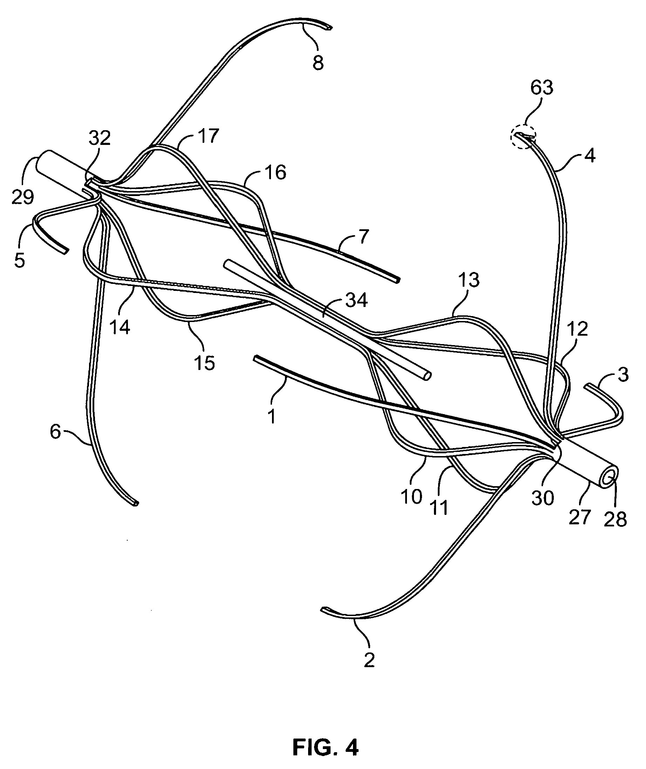

As shown in Figure 3b, the first set of expandable legs 1, 2, 3, 4 are secured at a point 30 which is proximal to end 28 on the second tube 27 . The second set of expandable legs 5, 6, 7, 8 are secured at a point 32 which is proximal to end 29 on the second tube 27. The length from point 30 to 28 and the length from point 32 to 29 range from about 2 mm to about 10 mm, from 3 mm to about 8 mm, from about 4 mm to about 7 mm or about 6 mm. The length from point 30 to 28 and the length from point 32 to 29 may be the same or be different. The free ends of the first set of expandable legs 1, 2, 3, 4 are in a direction opposite to the free ends of the second set of expandable legs 5, 6, 7 and 8. Each of the free ends of the first and second set of expandable legs 1, 2, 3, 4, and 5, 6, 7, 8 may have a barb 63 (Figure 3b). A solid pin or hollow tube 34 may be inserted in the second tube 27 and laser welded to the legs of the third and the fourth set of expandable legs (Figure 4). The length of the pin 34 may range from about 2 mm to about 8 mm, from about 3 mm to about 6 mm, or about 4 mm.

The first or second set of expandable legs may be secured at a point from about 2 mm to about 10 mm, from 3 mm to about 8 mm, from about 4 mm to about 7 mm or about 6 mm from the end of the second tube. The straight length of the third and the fourth set of expandable legs may be from about 5 mm to about 55 mm, from about 10 mm to about 50 mm, from about 20 mm to about 40 mm or about 30 mm.

Each slot of the first set 21 on the first tube 20 is positioned at a radial position allowing for deployment of each expandable leg of the first set, 1, 2, 3, 4. Each slot of the second set 22 on the first tube 20 is positioned at a radial position allowing for deployment of each expandable leg of the second set 5, 6, 7, 8. Each slot of the third set 23 on the first tube 20 is positioned at a radial position allowing for deployment of the expandable segment in each leg of the third set of expandable legs 10, 11, 12, 13. Each slot of the fourth set 24 is positioned at a radial position allowing for deployment of the expandable segment in each leg of the fourth set 14, 15, 16, 17. The external diameter of the second tube is less than the internal diameter of the first tube. The filter of the present invention is assembled by inserting the second tube 27 into the first tube 20. All the expandable legs on the second tube are straight, i.e., not expanded, during insertion.

The first and second sets of expandable legs on the second tube may have a length of about 10 mm to about 30 mm, from about 15 mm to about 25 mm or about 20 mm. The expandable legs of each set may have a width ranging from 0.05 mm to about 1.5 mm, from about 0.1 mm to about 1.0 mm, from about 0.3 mm to about 0.8 mm or about 0.35 mm. The width of the first and second sets of expandable legs may be constant or vary. For example, in one embodiment, the width of the first and second set of expandable legs may taper or narrow from the point where it is secured to the free end. The expanded diameter of the first and second set of expandable legs may range from 10 mm to about 45 mm, from about 15 mm to about 40 mm, from about 20 mm to about 36 mm or about 30 mm.

The dimensions of the second tube may vary. For example, the straight length of the second tube may range from about 25 mm to about 60 mm, from about 30 mm to about 50 mm or from about 35 mm to about 48 mm or about 45 mm. The external diameter of the second tube may vary from about 0.5 mm to about 1.5 mm, from about 0.8 mm to about 1.5 mm, from about 1.2 mm to about 1.5 mm, from 1.4 mm to about 1.5 mm or about 1.45 mm, provided that the external diameter of the second is less than the internal diameter of the first tube. The thickness of the second tube may range from about 0.3 mm to about 0.6 mm, from about 0.4 mm to about 0.5 mm or from about 0.4 mm to about 0.45 mm. The diameter of the first tube may be the same as the diameter of the second tube or may be different.

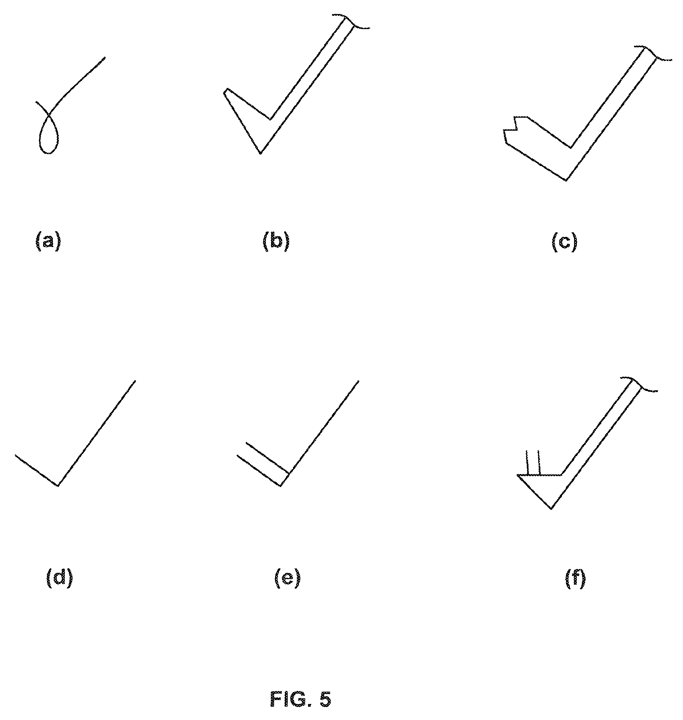

Each of the free ends of the first and the second sets of expandable legs may have at least one barb. The barbs may assume various designs and angles. For example, the angle between the barb and the free end of the leg where the barb is attached to may range from about 10 degrees to about 200 degrees, from about 40 degrees to about 200 degrees, from about 60 degrees to about 190 degrees, from about 90 degrees to about 180 degrees or from about 95 to about 105 degrees relative to a straight line set when the first set of expandable legs is in an undeployed position. The barbs can be any desired shape or configuration, the examples of which are shown in Figure 5. In one embodiment, the barb has a convex shape allowing it to bend on itself when the filter is deployed. The shape, configuration, dimension, angle and penetration depth of the barbs may vary between legs and may be present on some or all of the first and second set of expandable legs. In one embodiment, the angle of the barbs is set such that the barbs will not penetrate into the vessel wall until the internal vessel dimension of at least about 18 mm has been encountered by the filter. It will be appreciated that one of ordinary skill in the art could select both the shape and the angle of the barb by routine experimentation and that the shape of the cage formed by the first and second set of expandable legs can be constrained to meet this requirement.

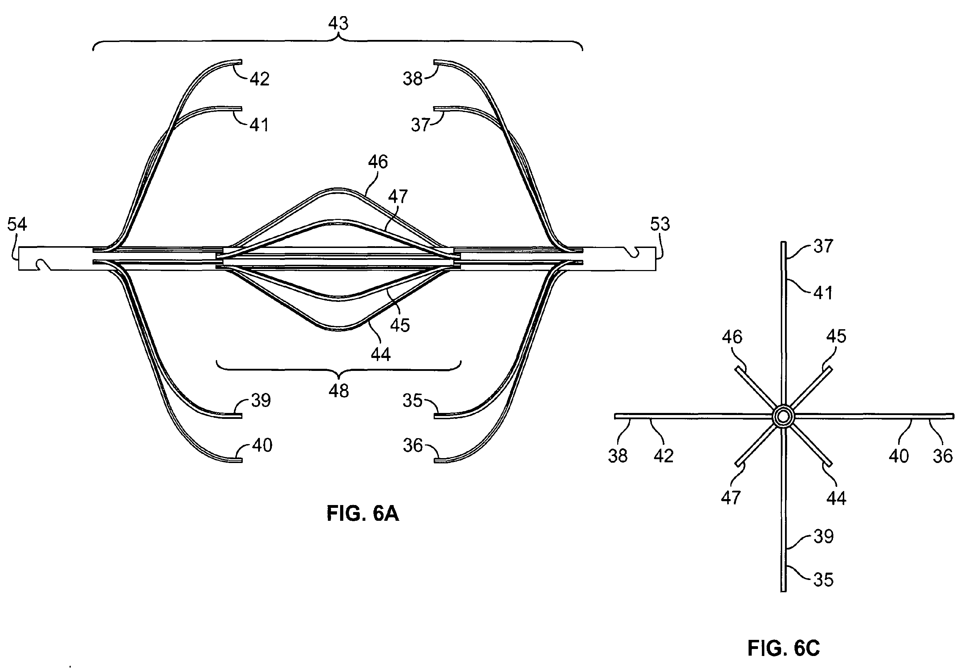

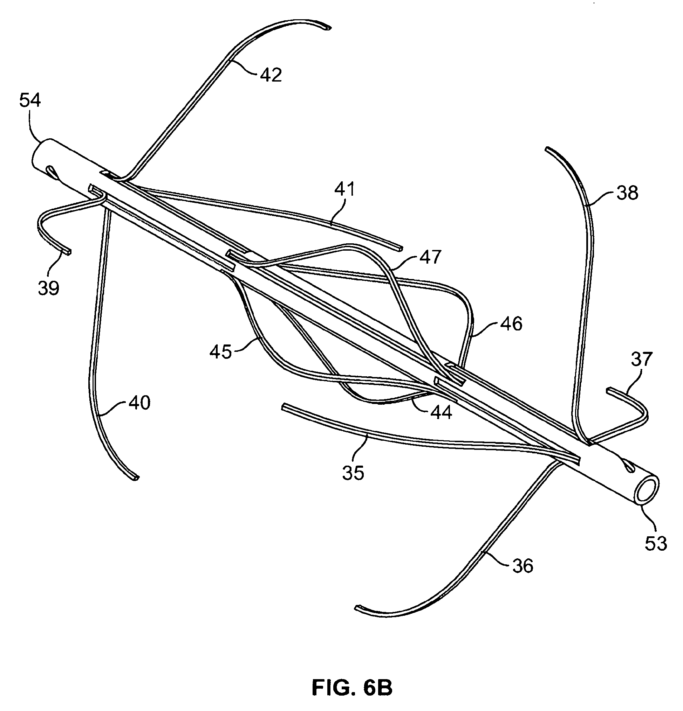

A second embodiment of the present filter is shown in Figures 6a, b, c. The filter comprises two tubes, a first tube and a second tube that is inserted into the first tube to form the filter. The external diameter of the second tube is smaller than the internal diameter of the first tube. The filter is formed from two sets of four expandable legs, the first expandable set, 35, 36, 37, 38 and the second expandable set, 39, 40, 41, 42. These expandable legs form a cage 43. The cage 43 may take the shape of a ball or sphere when deployed. The filter comprises a first set of expandable legs, 35, 36, 37, 38, a second set of expandable legs 39, 40, 41, 42 and a third set of expandable legs 44, 45, 46, 47. The expandable segments of the third set of expandable legs form a curvilinear shape 48 when deployed.

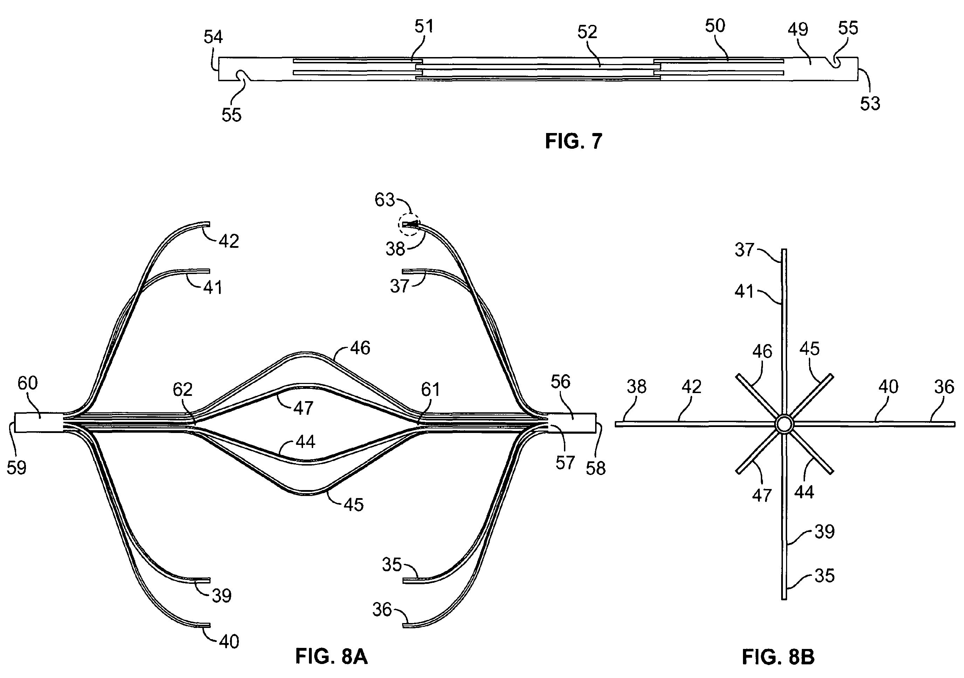

Figures 7 shows a perspective view of the first tube of the filter in Figure 6. The first tube 49 contains a plurality of a first set of slots 50, a plurality of a second set of slots 51 and a plurality of a third set of slots 52 which are parallel to the long or cylindrical axis of the first tube. The first set of slots 50 are positioned closest to the end 53 of the first tube 49. The second set of slots 51 is positioned closest to the end 54 of the first tube 49. There is one notch 55 at each end of the first tube. Each slot of the first and second sets may range in length from about 4 mm to about 35 mm, from about 10 mm to about 25 mm or from about 15 mm to about 20 mm or about 17 mm. The length of the first set of slots 50 and the second set of slots 51 may be the same or be different. The third set of slots may range in length from about 10 mm to about 55 mm, from about 20 mm to about 50 mm, from about 25 mm to about 40 mm, or about 30 mm.

Figures 8a and b show various perspective views of the second tube which comprises three sets of expandable legs. The first set of expandable legs 35, 36, 37, 38 are attached or secured to the second tube 56 at position 57. The second tube 56 has two ends 58, 59. The second set of expandable legs 39, 40, 41, 42 are attached or secured to the second tube 56 at position 60. The two ends of each expandable leg of the third set 44, 45, 46, 47 are attached to the second tube 56 at positions 61, 62. The dimension from point 57 to end 58, and from point 60 to end 59 may be from about 2 mm to about 10 mm, from about 3 mm to about 9 mm, from about 4 mm to about 8 mm or about 6 mm. The dimension from point 57 to end 58 and the dimension from point 60 to end 59 may be same or be different. The dimension from point 61 to 57 and the dimension from point 62 to 60 may be from about 0 mm to about 20 mm, from about 3 mm to about 15 mm, from about 5 mm to about 10 mm or about 8 mm. The dimension from point 61 to 57 and the dimension from point 62 to 60 may be the same or be different. The straight length of the third set of expandable legs may be from about 5 mm to about 55 mm, from about 10 mm to about 50 mm, from about 20 mm to about 40 mm or about 30 mm. In this embodiment, as is apparent from Figure 8b, the third set of expandable legs, 44, 45, 46, 47 are positioned at different radial points along the circumference of the second tube 56 as compared with the first set of expandable legs, 35, 36, 37, 38. The second set of expandable legs, 39, 40, 41, 42 may be positioned at the same radial points along the circumference of the second tube 56 as compared with the first set of expandable legs, 35, 36, 37, 38. The free ends of the first set of expandable legs 35, 36, 37, 38 are in a direction opposite to the free ends of the second set of expandable legs 39, 40, 41, 42. Each of the free ends of the first and the second set of expandable legs 35, 36, 37, 38, and 39, 40, 41, 42 may have a barb 63.

Each slot of the first set 50 on the first tube 49 is positioned at a radial position allowing for deployment of the first set of expendable legs, 35, 36, 37, 38. Each slot of the second set 51 on the first tube 49 is positioned at a radial position allowing for deployment of the second set of expendable legs 39, 40, 41, 42. Each slot of the third set 52 on the first tube 49 is positioned at a radial position for the deployment of the third set of expandable legs 44, 45, 46, 47.

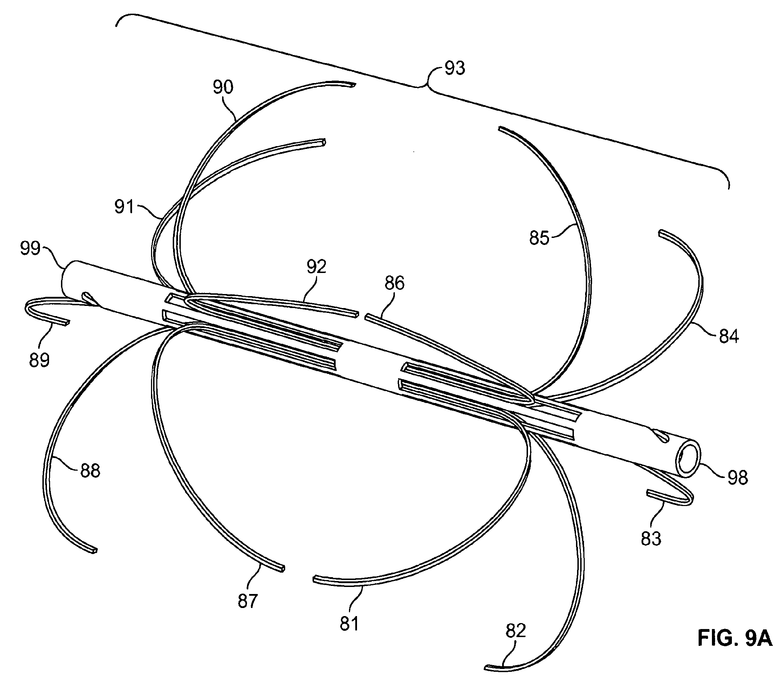

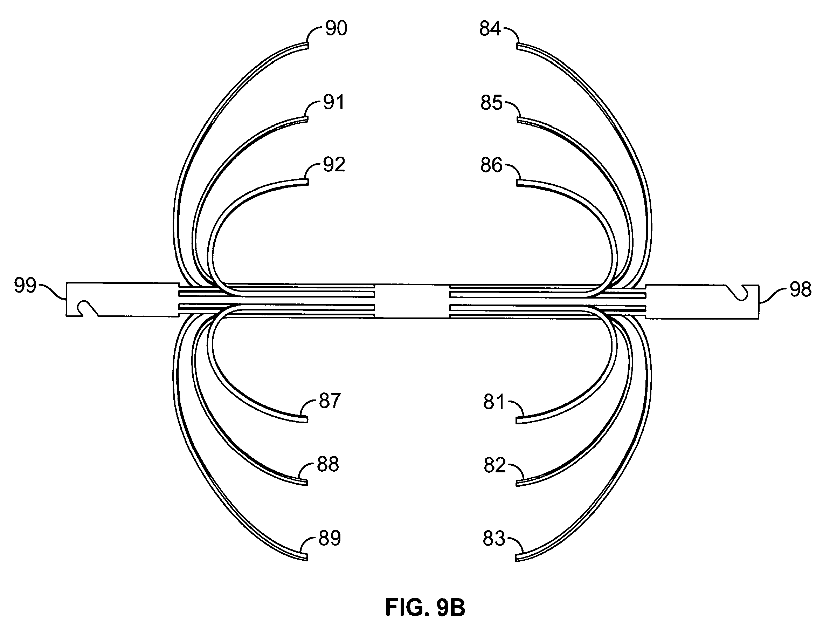

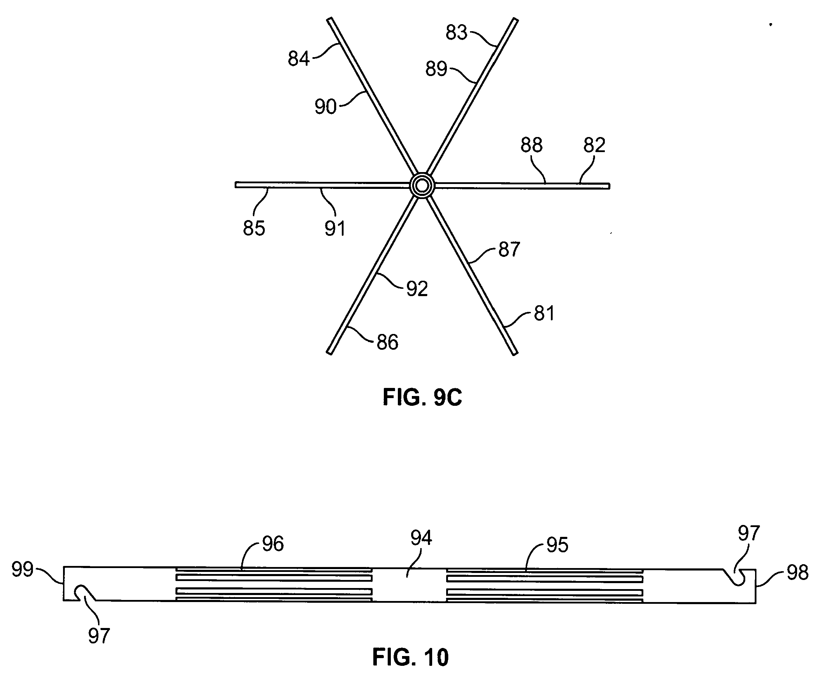

A filter not forming part of the invention is shown in Figures 9a, b, c. The filter comprises a first tube and a second tube that is inserted into the first tube. The external diameter of the second tube is smaller than the internal diameter of the first tube. The filter comprises a first set of expandable legs, 81, 82, 83, 84, 85, 86, a second set of expandable legs 87, 88, 89, 90, 91, 92. These expandable legs form a cage 93. The cage 93 may take the shape of a ball or sphere when deployed.

Figure 10 shows a perspective view of the first tube of the filter in Figure 9. The first tube 94 contains a plurality of a first set of slots 95, and a plurality of a second set of slots 96. The slots are parallel to the cylindrical axis of the first tube. The first set of slots 95 is positioned closest to the end 98 of the first tube 94. The second set of slots 96 is positioned closest to the end 99 of the first tube 94. There is one notch 97 at each end of the first tube. The lengths of the first set of slots 95 and the second set of slots 96 may be the same or be different.

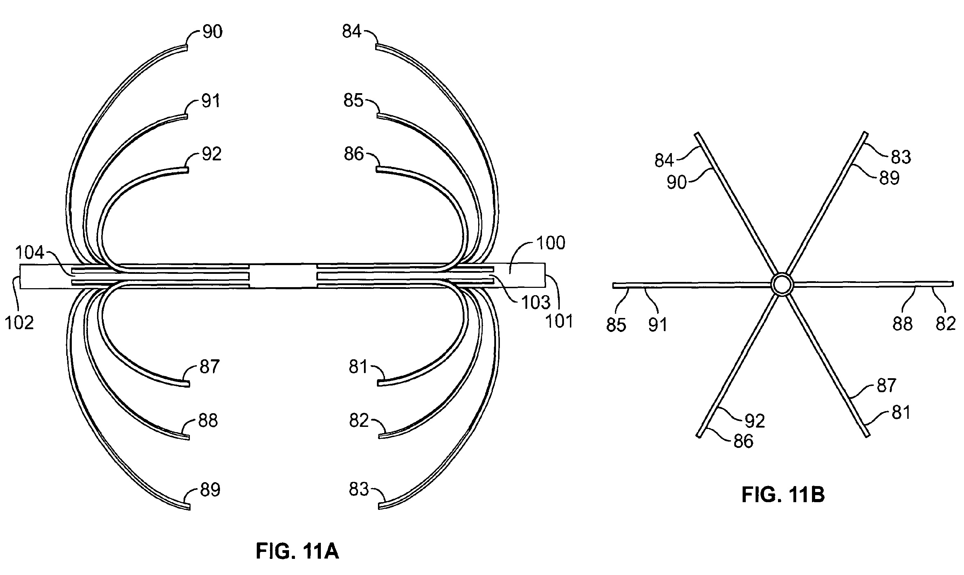

Figures 11 a and 11b show various perspective views of the second tube of the filter in Figure 9. The second tube 100 has two ends 101, 102. The second tube is hollow and comprises two sets of expandable legs. The first set of expandable legs 81, 82, 83, 84, 85, 86 are attached or secured to the second tube 100 at position 103. The second set of expandable legs 87, 88, 89, 90, 91, 92 are attached or secured to the second tube 100 at position 104. In this example, as is apparent from Figure 11b, the second set of expandable legs, 87, 88, 89, 90, 91, 92 are positioned at same radial points along the circumference of the second tube 100 as compared with the first set of expandable legs, 81, 82, 83, 84, 85, 86. The free ends of the first set of expandable legs 81, 82, 83, 84, 85, 86 are in a direction opposite to the free ends of the second set of expandable legs 87, 88, 89, 90, 91, 92. Each of the free ends of the first and the second set of expandable legs 81, 82, 83, 84, 85, 86, and 87, 88, 89, 90, 91, 92 may have a barb 63. Each slot of the first set 95 on the first tube 94 is positioned at a radial position allowing for deployment of the first set of expendable legs, 81, 82, 83, 84, 85, 86. Each slot of the second set 96 on the first tube 94 is positioned at a radial position allowing for deployment of the second set of expendable legs 87, 88, 89, 90, 91, 92.

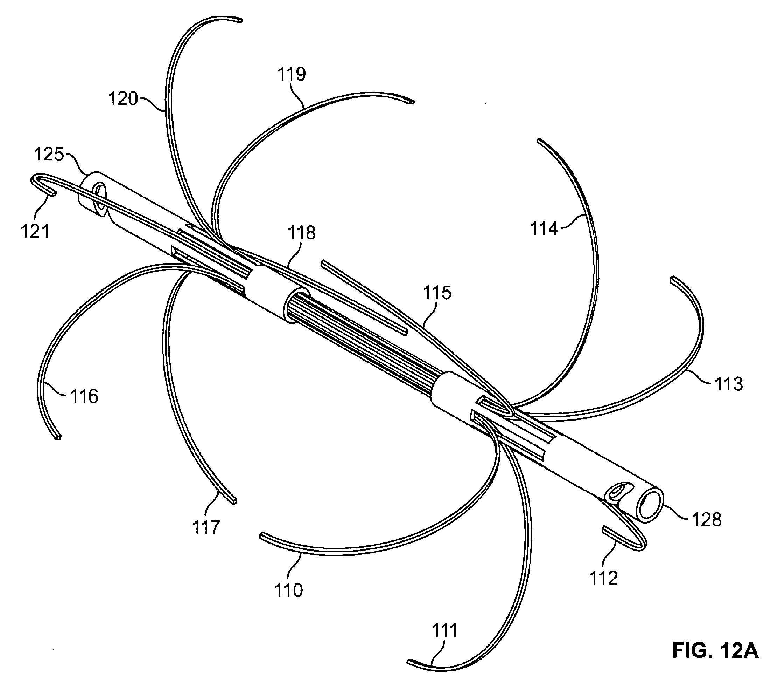

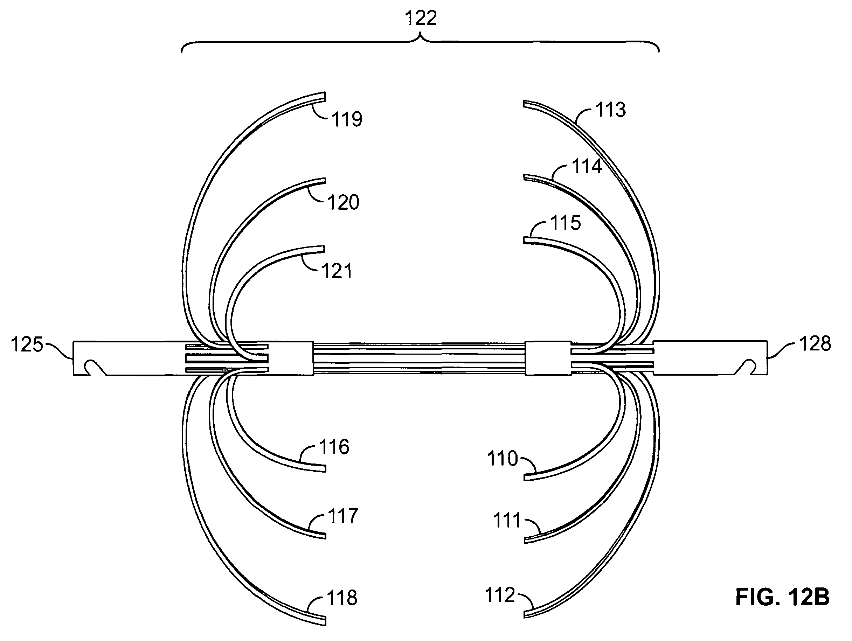

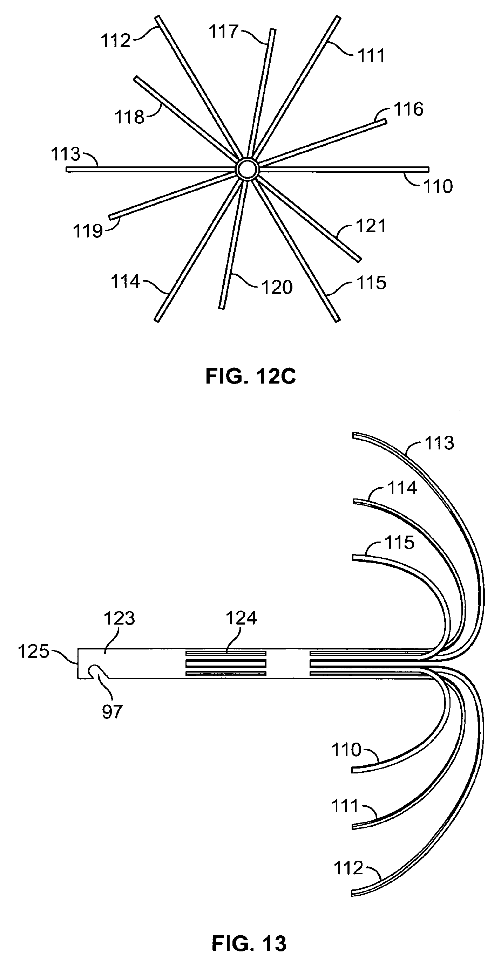

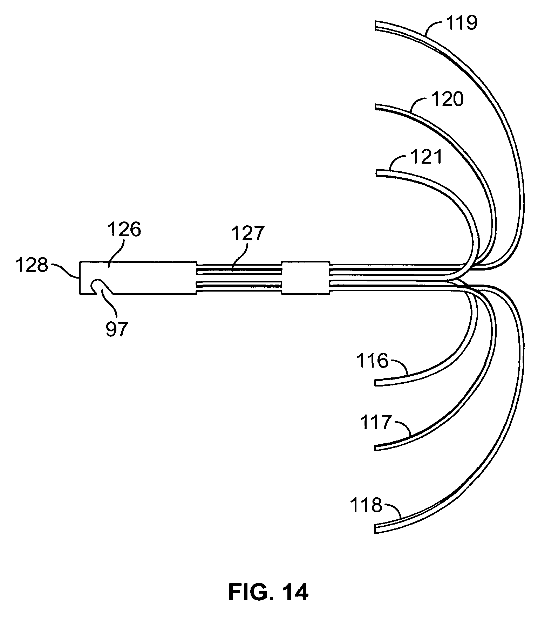

A filter not forming part of the invention is shown in Figures 12a, b, c. The filter is formed from two tubes with two sets of six expandable legs, the first expandable set, 110, 111, 112, 113, 114, 115, and the second expandable set, 116, 117, 118, 119, 120, 121. These expandable legs form a cage 122. The cage 122 may take the shape of a ball or sphere when deployed. Figures 13 shows a perspective view of the first tube of the filter in Figure 12. The first tube 123 contains a plurality of a first set of slots 124 which are parallel to the long or cylindrical axis of the first tube, and a plurality of a first set of expandable legs 110, 111, 112, 113, 114, 115. There is one notch 97 at the end 125 of the first tube 123. Figure 14 shows perspective view of the second tube. The second tube 126 contains a plurality of a second set of slots 127 which are parallel to the long or cylindrical axis of the second tube, and a plurality of a second set of expandable legs 116, 117, 118, 119, 120, 121. There is one notch 97 at the end 128 of the second tube 126. The length of the first set of slots 124 and the second set of slots 127 may be the same or be different. Each slot of the first set 124 on the first tube 123 is positioned at a radial position allowing for deployment of the second set of expendable legs, 116, 117, 118, 119, 120, 121 of the second tube 126. Each slot of the second set 127 on the second tube 126 is positioned at a radial position allowing for deployment of the first set of expendable legs 110, 111, 112, 113, 114, 115 of the first tube. The diameter of the first tube may be the same as the diameter of the second tube or may be different.

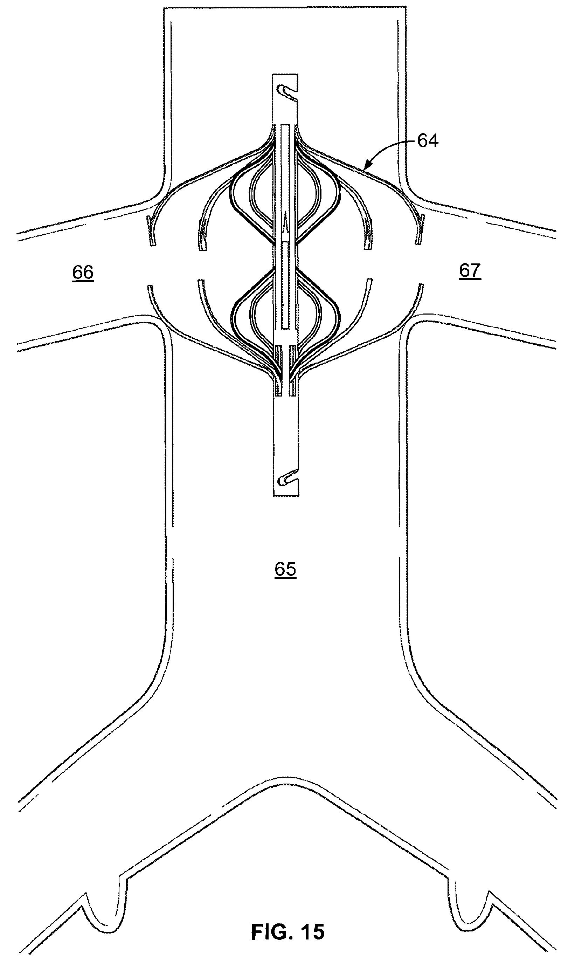

The filter of the present invention may be deployed by any desired delivery system. Figure 15 shows the filter 64 which is deployed in the inferior vena cava 65 at or below the junction of the right 66 and left 67 renal veins. The diameter of the axially collapsed filter may range from about 0.8 mm to about 5.5 mm, from about 1.2 mm to about 4.5 mm, or from about 1.5 mm to about 3 mm, with one specific embodiment of about 2 mm. The diameter of the delivery system, such as a delivery catheter, may range from about 0.8 mm to about 5.5 mm, from about 1.2 mm to about 4.5 mm, or from about 1.8 to about 3 mm. In one embodiment, the collapsed filter is encased in a delivery catheter of about 6 French (2 mm) in diameter. In another embodiment, the filter is delivered by a delivery catheter of about 8 French (2.67 mm) in diameter. The filter may be deployed by a simple pin and pull delivery (see Cordis TrapEase Permanent Vena Cava Filter with the VisEase Angiographic Vessel Dilator [on-line]. Retrieved on August 1, 2008, from URL: <http://www.mitek.com/home.jhtml?loc=USENG&page=viewContent&contentId=0900 8b9880ffdcbf&nodekey=/Prod_Info/Type/Endovascular_Disease_Management/Vena_Ca va_Filters&parentId=fcOde00100001215>. The filter may be placed in a vessel such as the inferior vena cava using ultrasound at a patient's bedside or under standard fluoroscopy. A catheter containing the undeployed filter is inserted and the filter extruded from the catheter. The filter then floats into place at or below the junction of the left and right renal vein. After insertion and deployment, the filter assumes a position within the inferior vena cava at or near the junction of the left and right renal veins 66, 67 (Figure 15). The first and second sets of expandable legs 1, 2, 3, 4 and 5, 6, 7, 8 form two hemispheres respectively in the vessel creating a cage or sphere 9. The barbs of the filter open and insert into the vessel wall when the width of the vessel wall exceeds the diameter of the filter. The barbs are on the ends of the legs which are on the outer curvature of the spheres. The sphere shape prevents barb deployment until the diameter of the blood vessel exceeds the deployment diameter for the barbs. The deployment diameter for the barbs may range from about 7 mm to about 20 mm, from about 10 mm to about 18 mm or about 15 mm.

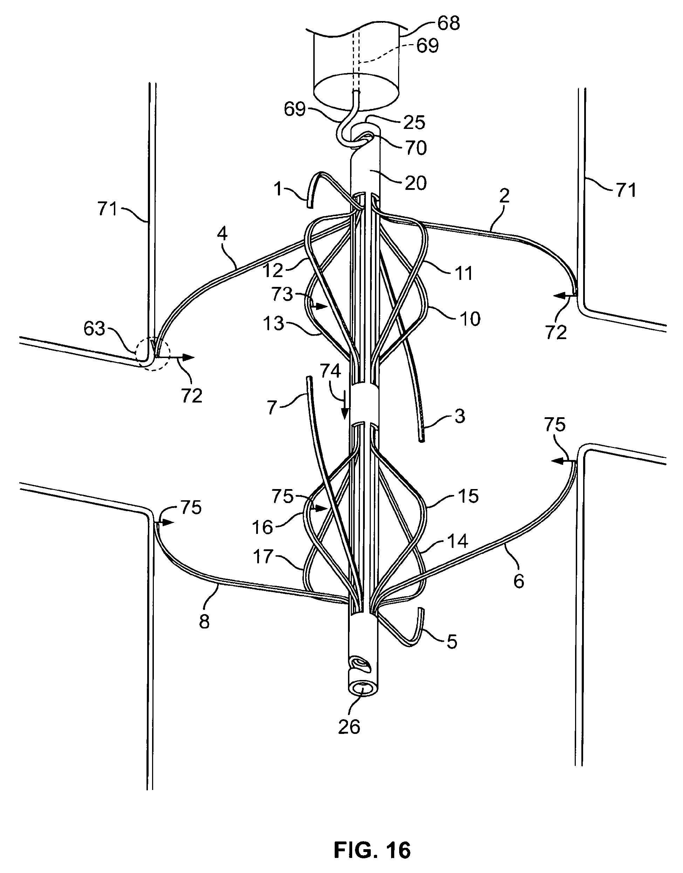

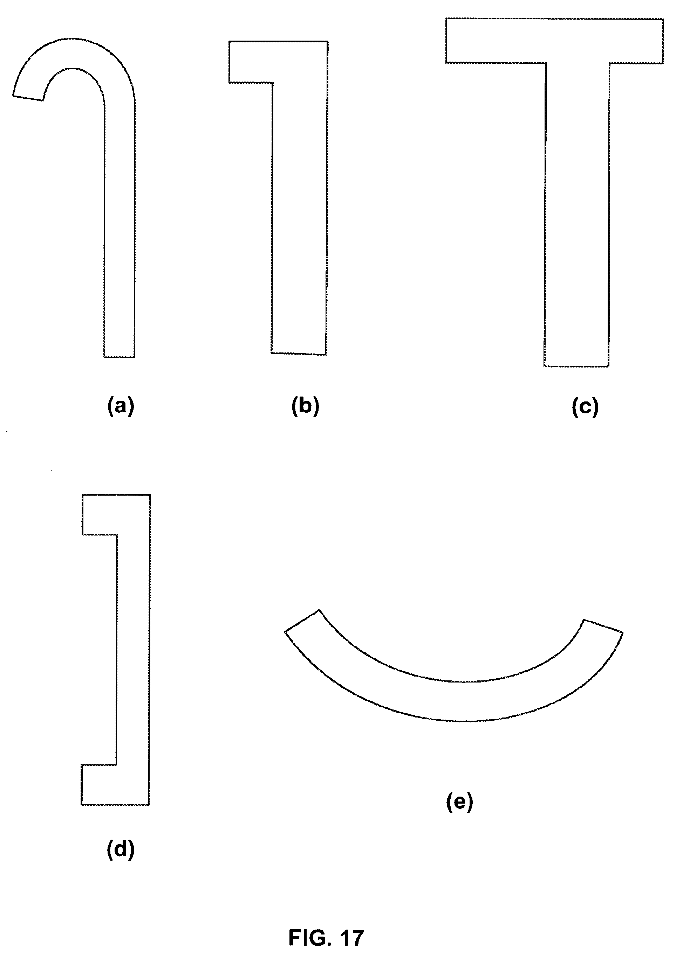

A method for retrieving the vena cava filter is disclosed as an example. Figure 16 illustrates retrieval of the filter shown in Figure 1. A catheter 68, the internal dimension of which is greater than the external dimension of the first tube 20, is inserted into a vessel such as the jugular vein or femoral vein and moved to where the filter is positioned on the vessel wall. A snare 69 is pushed through the catheter until the snare grabs the notch 70 which is present on the first tube 20. The notch 70 is shown in this embodiment as a semicircular structure. The notch may have a variety of different structures such as a rectangular hole or a square hole as long as it permits efficient hooking of the filter 64 by a snare 69. The notch 70 may be positioned from about 2 mm to about 10 mm from the end 25, from about 4 mm to about 8 mm, from about 6 mm to about 8 mm and about 5 mm. The physician exerts tension on the filter 64 by pulling back on the snare 69. The tension exerted may be in the range of about 0.45 kilogram (kg) to about 5 kg, but the appropriate amount of tension may be determined by one of ordinary skill in the art based on clinical experience in the art. Various embodiments of the notch 70 are shown in Figure 17 ((a) - (e)). The notch 70 may assume many different shapes such as a hook (a), an L-shape (b), a T-shape (c), a reverse C-shape (d) and a semi-circular shape (e) as long as it permits secure capture by the snare 69. The snare may also take many different forms, such as a loop or a wire basket. The snare may be formed from several interconnected pieces of material or from a single piece of material. In addition, the snare may comprise a locking mechanism that locks once the snare grabs the notch on the filter. The catheter 68 is pushed over the snare, each leg of the first set of expandable legs 1, 2, 3, 4 and each leg of the third set of expandable legs 10, 11, 12, 13 until the barbs 63 dislodge from the vessel wall, each expandable leg of the first set 1, 2, 3, 4 retracts from the wall 71 of vena cava and move inwards 72. Meanwhile, as the catheter is pushed over the third set of expandable legs 10, 11, 12, 13, each expandable segment of the third set of expandable legs 10, 11, 12, 13 straightens 73 and pushes downwards away from the snare 69. Straightening of the third set of expandable legs creates a vector of force 74 which pushes the second tube 20 away from the snare 69 further creating a vector of force 75. The vector of force 75 straightens the expandable segment of the fourth set of expandable legs 14, 15, 16, 17 and also pushes the second set of expandable legs away from the vessel wall 71. The catheter 68, which encompasses the retracted filter, is then withdrawn from the vessel.

The filter may also be retrieved from the other end 26 of the filter (Figure 16). The method of retrieving the filter from the other end 26 is similar as set forth except that the catheter is pushed over the second set 5, 6, 7, 8 and fourth set 14, 15, 16, 17 of expandable legs. The second set of expandable legs 5, 6, 7, 8 retract from the vessel wall, and the fourth set of expandable legs 14, 15, 16, 17 straighten. Straightening of the fourth set of expandable legs pushes the second tube away from end 26, straightens the third set of expandable legs 10, 11, 12, 13, and pushes the first set of expandable legs 1, 2, 3, 4 away from the vessel wall.

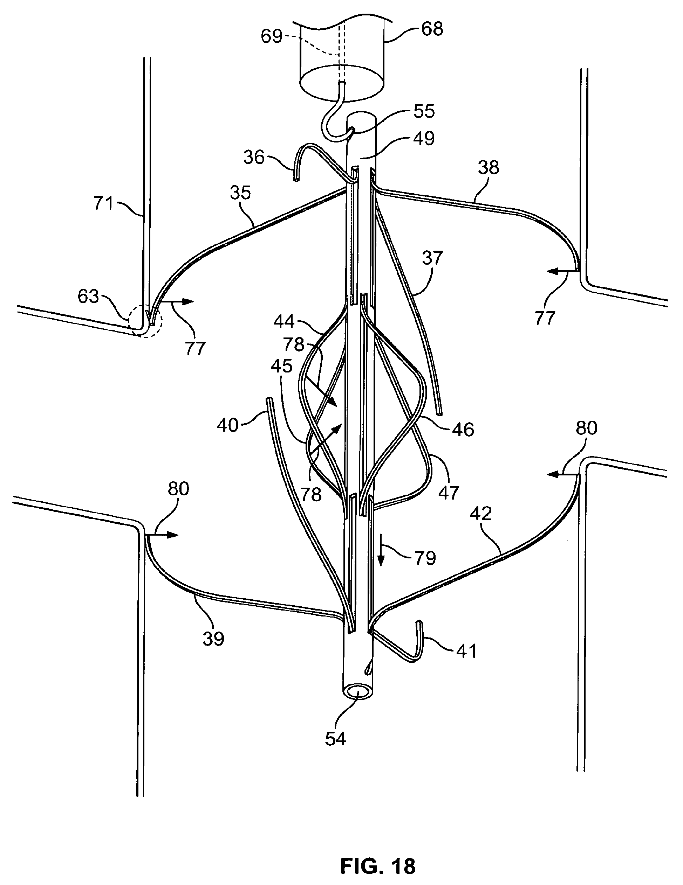

Figure 18 illustrates retrieval of the filter shown in Figure 6. A catheter 68, the internal dimension of which is greater than the external dimension of the first tube 49, is inserted into a vessel such as the jugular vein or femoral vein and moved to where the filter is positioned on the vessel wall. A snare 69 is pushed through the catheter 68 until the snare grabs the notch 55. A notch structure is present on the first tube 49. The physician exerts tension on the filter by pulling back on the snare 69. The tension exerted may be in the range of about 0.45 kilogram (kg) to about 5 kg, but the appropriate amount of tension may be determined by one of ordinary skill in the art based on clinical experience in the art. The catheter 68 is pushed over the snare 69 and each leg of the first set of expandable legs 35, 36, 37, 38 until each expandable leg retracts from the wall 71 of vena cava. The barbs 63 dislodge from the vessel wall and move inwards 77. As each leg of the first set 35, 36, 37, 38 of expandable legs retracts from the vessel wall, the catheter 68 further pushes over the expandable segments of the third set of expandable legs 44, 45, 46, 47 causing the expandable segments of the third set of expandable legs straighten 78 and push downwards away from the snare 69. Straightening of the expandable legs 44, 45, 46, 47 generates a vector of force 79 which pushes the second tube 49 away from the snare 69 creating a vector force 80 which pushes the second set of expandable legs 39, 40, 41, 42 away from the vessel wall 71. The catheter 68, which encompasses the retracted filter, is then withdrawn from the vessel.

The filter may be retrieved from the other end 54 of the filter (Figure 18). The method of retrieving the filter from the other end 54 is similar as set forth except that the catheter is pushed over the second set 39, 40, 41, 42 of expandable legs. The second set of expandable legs 39, 40, 41, 42 retract from the vessel wall, and the third set of expandable legs 44, 45, 46, 47 straighten. Straightening of the third set of expandable legs pushes the second tube away from end 54, and the first set of expandable legs 35, 36, 37, 38 away from the vessel wall.

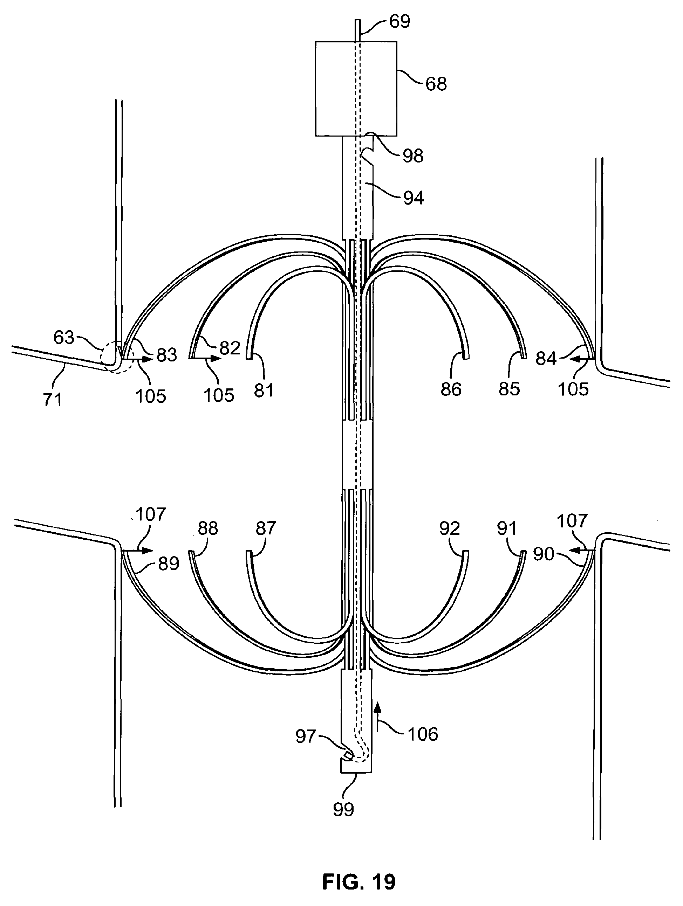

Figure 19 illustrates retrieval of the filter shown in Figure 9. A catheter 68, the internal dimension of which is greater than the external dimension of the first tube 94, is inserted into a vessel such as the jugular vein or femoral vein and moved to where the filter is positioned on the vessel wall. A snare 69 is pushed through the catheter 68 and the inner space of the second tube until the snare grabs the notch 97 closest to the end 99. The physician exerts tension on the filter by pulling back on the snare 69 and pushing the catheter 68. The catheter 68 is pushed over each leg of the first set of expandable legs 81, 82, 83, 84, 85, 86 until each expandable leg retracts from the wall 71 of vena cava. The barbs 63 dislodge from the vessel wall and move inwards 105. As the snare 69 is pulled back, the first tube is pulled towards 106 the catheter, exerting tension on the second set of expandable legs 87, 88, 89, 90, 91, 92, causing the second set of expandable legs 87, 88, 89, 90, 91, 92 to move inwards 107, and retract from the vessel wall 71. The catheter 68, which encompasses the retracted filter, is then withdrawn from the vessel.

The filter may be retrieved from the other end 99 of the filter (Figure 19). The method of retrieving the filter from the other end 99 is similar as set forth except that a snare 69 is pushed through the catheter 68 and the second tube until the snare grabs the notch 97 closest to the end 98. The catheter is pushed over the second set 87, 88, 89, 90, 91, 92 of expandable legs. The second set of expandable legs 87, 88, 89, 90, 91, 92 retract from the vessel wall. The snare pulls back the first tube pushing the first set of expandable legs 81, 82, 83, 84, 95, 86 and the first set of expandable legs 81, 82, 83, 84, 85, 86 retract from the vessel wall. The filter shown in Figure 12 may be retrieved using a similar mechanism as shown in Figure 19.

The filter may be retrieved by the use of a snare and a catheter, or may be retrieved using a catheter and a ratchet control mechanism to control the catheter.

The filter may be made of laser cut, self-expanding nitinol. The filter may also be made of any metal, such as titanium, platinum, gold, a metal alloy, such as stainless steel, or a memory metal. In one embodiment, each of the expandable legs of the first and second sets comprise memory metal. In another embodiment, the expandable segment of the expandable legs of the third and the fourth set comprises memory metal. The filter may further be made of any biocompatible material that is durable and non-corrosive. Examples of biocompatible material include a synthetic material such as polyurethanes, segmented polyurethane-urea/heparin, poly-L-lactic acid, cellulose ester, polyethylene glycol, polyvinyl acetate, dextran and gelatin, a naturally-occurring material such as basement membrane components such as collagen, elastin, laminin, fibronectin, vitronectin; fibrin, cellulose, and amorphous carbon, or fullerenes. In some embodiments of the invention, the filter is made of biodegradable, bioabsorbable, bioerodable material and/or a mixture thereof. Examples of bioabsorbable material include copolymers of glycolide with lactide or ε-caprolactone, and poly(p-dixanone). The filter may be made of a single material or different materials. U.S. Patent Publication No. 20070191932. U.S. Patent No. 7,147,649.

The filter of the present invention may be manufactured in numerous ways. The filter may be formed from a single piece of material by removing various portions of a tube or pipe's wall to form the configurations described herein in an example not forming part of the invention. The filter is manufactured by connecting various segments together. Material from the tube wall may be removed using various techniques including laser (e.g. YAG laser), electrical discharge machining, mechanical machining, chemical etching (e.g. photo-fabrication), metal cutting, a combination thereof, or other well known techniques. See U.S. Patent No. 7,329,277, U.S. Patent No. 5,879,381, and U.S. Patent No. 6,117,165.

While the vena cava filters are preferred embodiments of the present invention, filters within the scope of the invention may be placed in any desired blood vessel or endovascular structure. The filter may be placed via a femoral access point, jugular access point or any desired intravascular route. The filter may be placed in the body of the patient permanently or temporarily before being retrieved.

After the vena cava filter is deployed, the vascular endothelial cells or other tissues grow where the filter and vessel wall contact. When the filter is retrieved later, severe damage may occur resulting in laceration or rupture of the vena cava, or at the very least, a focal disruption of the endothelial lining which may predispose to caval stenosis, thrombosis or occlusion. To reduce the risk of complications, the free ends of the legs, other parts of the filter, or the entire filter can be coated with an antiproliferative agent to prohibit the tissue ingrowth, an anti-inflammatory agent or any desired pharmaceutically active agents. Examples of anti-proliferative agents include paclitaxel (taxol), paclitaxel derivatives, rapamycin (sirolimus), rapamycin derivatives (including everolimus, zotarolimus, biolimus and biolimus A-9), 5-fluorouracil, cisplatin, vinblastine, vincristine, epothilones, endostatin, angiostatin and thymidine kinase inhibitors or a combination thereof. Examples of the anti-inflammatory agent include dexamethasone, corticosterone and prednisolone. Examples of pharmaceutically active agents that prevent or reduce thrombus formation include anticoagulants, antiplatelets and fibrinolytics. The pharmaceutically active agents include therapeutic agents and/or diagnostic agents. The therapeutic agents may comprise drugs that are used in the treatment of vascular disease, including artherosclerosis, restenosis, thrombosis. The pharmaceutically active agent may be antibiotics/antimicrobials, antiproliferatives, antineoplastics, antioxidants, endothelial cell growth factors, thrombin inhibitors, immunosuppressants, anti-platelet aggregation agents, collagen synthesis inhibitors, therapeutic antibodies, nitric oxide donors, antisense oligonucleotides, wound healing agents, therapeutic gene transfer constructs, peptides, proteins, extracellular matrix components, vasodialators, thrombolytics, anti-metabolites, growth factor agonists, antimitotics, stating, steroids, steroidal and non-steroidal anti-inflammatory agents, angiotensin converting enzyme (ACE) inhibitors, free radical scavengers, PPAR-gamma agonists and anti-cancer chemotherapeutic agents. Examples of the pharmaceutically active agents in the present invention also include rosuvastatin, cyclosporin A (CSA), mycophenolic acid (MPA), retinoic acid, n-butyric acid, butyric acid derivatives, vitamin E, probucol, L-arginine-L- glutamate, tacrolimus (FK-506), puerarin, platelet factor 4, basic fibroblast growth factor (bFGF), fibronectin, simvastatin, fluvastatin, ABT-578, interferon, dexamethasone, dihydroepiandrosterone (DHEA) and estradiol.

Any part of the filter or the entire filter can be coated with any desired pharmaceutically active agents. An excipient may be coated on the filter together with the pharmaceutically active agent. The examples of the excipient include binder, matrix, carrier, polymer, hydrogel and nanoparticle. The coating on the filter may be smooth, semi-porous or porous. The coating may be one layer or multiple layers. A pharmaceutically active agent or excipient may also be deposited in a defined structure of the filter, such as tubes, grooves, wells, bells, baskets, etc. A pharmaceutically active agent may also be incorporated into a biocompatible polymer matrix. Polymer matrices include polymers such as poly(lactide-co-glycolide); poly-DL-lactide, poly-L-lactide, and/or mixtures thereof and can be of various inherent viscosities and molecular weights. In one embodiment, poly(DL lactide-co-glycolide) (DLPLG, Birmingham Polymers Inc.) can be used. U.S. Patent Publication No. 20070141107. The pharmaceutically active agent may be released in a sustained, delayed, spiked, controlled or any desired manner.

The scope of the present invention is not limited by what has been specifically shown and described hereinabove. Those skilled in the art will recognize that there are suitable alternatives to the depicted examples of materials, configurations, constructions and dimensions. Numerous references, including patents and various publications, are cited and discussed in the description of this invention. The citation and discussion of such references is provided merely to clarify the description of the present invention and is not an admission that any reference is prior art to the invention described herein. Variations, modifications and other implementations of what is described herein will occur to those of ordinary skill in the art without departing from the scope of the appended claims. While certain embodiments of the present invention have been shown and described, it will be obvious to those skilled in the art that changes and modifications may be made without departing from the scope of the appended claims. The matter set forth in the foregoing description and accompanying drawings is offered by way of illustration only and not as a limitation.

## Claims

1. A filter comprising,
a first tube (20, 49) having a plurality of a first set of slots (21, 50),
a second tube (27, 56) having a plurality of a first set of expandable legs (1, 2, 3, 4, 35, 36, 37, 38),
each leg of the first set having an end secured to the second tube and a free end,
each slot of the first set of slots on the first tube being positioned at a radial position allowing for deployment of each expandable leg of the first set,
**characterized in that**;
the first tube has a plurality of a second set of slots (22, 51) and a plurality of a third set of slots (23, 52),
the second tube has a plurality of a second set of expandable legs (5, 6, 7, 8, 39, 40, 41, 42) and a plurality of a third set of expandable legs (10, 11, 12, 13, 44, 45, 46, 47),
each leg of the second set has an end secured to the second tube and a free end, the free end of each leg in the first set being oriented in a direction opposite to the free end of each leg in the second set,
each leg of the third set comprises an expandable segment and has both ends secured to the second tube,
each slot of the second set of slots on the first tube is positioned at a radial position allowing for deployment of each leg of the second set, and each slot of the third set of slots on the first tube is positioned at a radial position allowing for deployment of each leg of the third set, the slots being oriented parallel to the cylindrical axis of the first tube,
wherein the second tube's external diameter is less than the first tube's internal diameter, and wherein the second tube is inserted into the first tube.

2. The filter of claim 1, further comprising a plurality of a fourth set of slots (24) on the first tube and a plurality of a fourth set of expandable legs (14, 15, 16,17) on the second tube, each leg of the fourth set comprising an expandable segment and having both ends secured to the second tube, wherein each slot of the fourth set of slots is positioned at a radial position allowing for deployment of each leg of the fourth set of expandable legs, the slots being orientated parallel to the cylindrical axis of the first tube.

3. The filter of either of the preceding claims wherein a cage (9, 43) is formed comprising expandable legs from the first and second sets.

4. The filter of any of the preceding claims, wherein each expandable leg of the first set and the second set has at least one barb (63) on the free end.

5. The filter of claims 1 or 2 wherein
i) the expandable segments of the third set or
ii) the expandable segments of the third set and the expandable segments of the fourth set;
each form(s) a curvilinear shape when deployed.

6. The filter of claims 1 or 2 wherein a segment of each expandable leg of the third set and/or the fourth set is secured to a pin (34) or tube (34) which lies within the second tube.

7. The filter of any of the preceding claims wherein one end (25, 26, 53, 54) of the first tube and/or one end of the second tube has at least one notch (55).

8. The filter of any of the preceding claims wherein the number of expandable legs in each set is three, four, five or six and may be equal or unequal.

9. The filter of any of the preceding claims wherein the expandable legs are secured at symmetric radial positions along the circumference of the second tube.

10. The filter according to any of the preceding claims wherein the radial positions of one set of expandable legs is offset from the radial position of a different set of expandable legs.

11. The filter according to any of the preceding claims, wherein the filter is encased in a catheter in an undeployed state.

12. A tube as defined in any of claims 1 to 10 which is the second tube.

## Patentansprüche

1. Filter, aufweisend:
eine erste Röhre (20, 49), die eine Mehrzahl eines ersten Satzes von Schlitzen (21, 50) aufweist,
eine zweite Röhre (27, 56), die eine Mehrzahl eines ersten Satzes von spreizbaren Füße (1, 2, 3, 4, 35, 36, 37, 38) aufweist,
wobei jeder Fuß des ersten Satzes ein an der zweiten Röhre befestigtes Ende und ein freies Ende aufweist,
wobei jeder Schlitz des ersten Satzes von Schlitzen an der ersten Röhre bei einer Radialposition positioniert ist, die ein Entfalten eines jeden spreizbaren Fußes des ersten Satzes zulässt,
**dadurch gekennzeichnet, dass**:
die erste Röhre eine Mehrzahl eines zweiten Satzes von Schlitzen (22, 51) und eine Mehrzahl eines dritten Satzes von Schlitzen (23, 52) aufweist,
die zweite Röhre eine Mehrzahl eines zweiten Satzes von spreizbaren Füßen (5, 6, 7, 8, 39, 40, 41, 42) und eine Mehrzahl eines dritten Satzes von spreizbaren Füßen (10, 11, 12, 13, 44, 45, 46, 47) aufweist,
wobei jeder Fuß des zweiten Satzes ein an der zweiten Röhre befestigtes Ende und ein freies Ende aufweist, wobei das freie Ende eines jeden Fußes in dem ersten Satz in einer Richtung entgegengesetzt zum freien Ende eines jeden Fußes in dem zweiten Satz ausgerichtet ist,
wobei jeder Fuß des dritten Satzes ein spreizbares Segment aufweist und seine beiden Enden an der zweiten Röhre befestigt sind,
wobei jeder Schlitz des zweiten Satzes von Schlitzen an der ersten Röhre bei einer Radialposition positioniert ist, die ein Entfalten eines jeden Fußes des zweiten Satzes zulässt, und jeder Schlitz des dritten Satzes von Schlitzen an der ersten Röhre bei einer Radialposition positioniert ist, die ein Entfalten eines jeden Fußes des dritten Satzes zulässt, wobei die Schlitze parallel zur Zylinderachse der ersten Röhre orientiert sind,
wobei der Außendurchmesser der zweiten Röhre geringer als der Innendurchmesser der ersten Röhre ist und wobei die zweite Röhre in die erste Röhre eingeführt ist.

2. Filter nach Anspruch 1, weiter aufweisend eine Mehrzahl eines vierten Satzes von Schlitzen (24) an der ersten Röhre und eine Mehrzahl eines vierten Satzes von spreizbaren Füßen (14, 15, 16, 17) an der zweiten Röhre, wobei jeder Fuß des vierten Satzes ein spreizbares Segment aufweist und seine beiden Enden an der zweiten Röhre befestigt sind, wobei jeder Schlitz des vierten Satzes von Schlitzen an der ersten Röhre bei einer Radialposition positioniert ist, die ein Entfalten eines jeden Fußes des vierten Satzes von spreizbaren Füßen zulässt, wobei die Schlitze parallel zur Zylinderachse der ersten Röhre orientiert sind.

3. Filter nach einem der vorhergehenden Ansprüche, wobei ein Käfig (9, 43), der spreizbare Füße aufweist, aus den ersten und zweiten Sätzen ausgebildet ist.

4. Filter nach einem der vorhergehenden Ansprüche, wobei jeder spreizbare Fuß des ersten Satzes und des zweiten Satzes mindestens einen Stachel (63) an dem freien Ende aufweist.

5. Filter nach Anspruch 1 oder 2, wobei
i) die spreizbaren Segmente des dritten Satzes oder
ii) die spreizbaren Segmente des dritten Satzes und die spreizbaren Segmente des vierten Satzes:
jeweils eine gekrümmte Form bilden, wenn sie entfaltet sind.

6. Filter nach Anspruch 1 oder 2, wobei ein Segment eines jeden spreizbaren Fußes des dritten Satzes und/oder des vierten Satzes an einem Stift (34) oder einer Röhre (34) befestigt ist, der/die innerhalb der zweiten Röhre liegt.

7. Filter nach einem der vorhergehenden Ansprüche, wobei das eine Ende (25, 26, 53, 54) der ersten Röhre und/oder das eine Ende der zweiten Röhre mindestens eine Einkerbung (55) aufweist.

8. Filter nach einem der vorhergehenden Ansprüche, wobei die Anzahl an spreizbaren Füßen in jedem Satz drei, vier, fünf oder sechs betragen kann und gleich oder ungleich sein kann.

9. Filter nach einem der vorhergehenden Ansprüche, wobei die spreizbaren Füße bei symmetrischen Radialpositionen entlang dem Umfang der zweiten Röhre befestigt sind.

10. Filter nach einem der vorhergehenden Ansprüche, wobei die Radialpositionen des einen Satzes von spreizbaren Füßen gegen die Radialposition eines unterschiedlichen Satzes von spreizbaren Füßen versetzt sind.

11. Filter nach einem der vorhergehenden Ansprüche, wobei das Filter in einem nicht-entfalteten Zustand in einen Katheter eingeschlossen ist.

12. Röhre wie definiert in einem der Ansprüche 1 bis 10, welche die zweite Röhre ist.

## Revendications

1. Filtre comprenant,
un premier tube (20, 49) ayant plusieurs premiers ensembles de fentes (21, 50),
un second tube (27, 56) ayant plusieurs premiers ensembles de pattes extensibles (1, 2, 3, 4, 35, 36, 37, 38),
chaque patte du premier ensemble ayant une extrémité fixée sur le second tube et une extrémité libre,
chaque fente du premier ensemble de fentes du premier tube étant positionnée dans une position radiale qui permet le déploiement de chaque patte extensible du premier ensemble, **caractérisé en ce que** :
le premier tube comporte plusieurs deuxièmes ensembles de fentes (22, 51) et plusieurs troisièmes ensembles de fentes (23, 52),
le second tube comporte plusieurs deuxièmes ensembles de pattes extensibles (5, 6, 7, 8, 39, 40, 41, 42) et plusieurs troisièmes ensembles de pattes extensibles (10, 11, 12, 13, 44 , 45, 46, 47),
chaque patte du deuxième ensemble a une extrémité fixée sur le second tube et une extrémité libre, l'extrémité libre de chaque patte du premier ensemble étant orientée dans une direction opposée à l'extrémité libre de chaque patte du deuxième ensemble,
chaque patte du troisième ensemble comprend un segment extensible et a deux extrémités solidaires fixées sur le second tube,
chaque fente du deuxième ensemble de fentes sur le premier tube est positionnée dans une position radiale qui permet le déploiement de chaque patte du deuxième ensemble, et
chaque fente du troisième ensemble de fentes sur le premier tube est positionnée dans une position radiale permettant le déploiement de chaque patte du troisième ensemble, les fentes étant orientées parallèlement à l'axe cylindrique du premier tube,
dans lequel le diamètre externe du second tube est inférieur au diamètre interne du premier tube, et dans lequel le second tube est inséré dans le premier tube.

2. Filtre selon la revendication 1, comprenant en outre plusieurs quatrièmes ensembles de fentes (24) sur le premier tube et plusieurs quatrièmes ensembles de pattes extensibles (14, 15, 16, 17) sur le second tube, chaque patte du quatrième ensemble comprenant un segment extensible et ayant deux extrémités fixées sur le second tube, dans lequel chaque fente du quatrième ensemble de fentes est positionnée dans une position radiale qui permet le déploiement de chaque patte du quatrième ensemble de pattes expansibles, les fentes étant orientées parallèlement à l'axe cylindrique du premier tube.

3. Filtre selon l'une quelconque des revendications précédentes, dans lequel une cage (9, 43) est formée comprenant des pattes extensibles des premiers et deuxièmes ensembles.

4. Filtre selon l'une quelconque des revendications précédentes, dans lequel chaque patte extensible du premier ensemble et du deuxième ensemble comporte au moins un ardillon (63) sur l'extrémité libre

5. Filtre selon les revendications 1 ou 2, dans lequel
i) les segments extensibles du troisième ensemble ou
ii) les segments extensibles du troisième ensemble et les segments expansibles du quatrième ensemble ;
forment chacun une forme curviligne lorsqu'ils sont déployés.

6. Filtre selon les revendications 1 ou 2, dans lequel un segment de chaque patte extensible du troisième ensemble et/ou du quatrième ensemble est fixée sur une broche (34) ou un tube (34) qui se trouve à l'intérieur du second tube.

7. Filtre selon l'une quelconque des revendications précédentes, dans lequel une extrémité (25, 26, 53, 54) du premier tube et/ou une extrémité du second tube comporte au moins une encoche (55).

8. Filtre selon l'une quelconque des revendications précédentes, dans lequel le nombre de pattes extensibles dans chaque ensemble est de trois, quatre, cinq ou six et peut être égal ou différent.

9. Filtre selon l'une quelconque des revendications précédentes, dans lequel les pattes extensibles sont fixées dans des positions radiales symétriques le long de la circonférence du second tube.

10. Filtre selon l'une quelconque des revendications précédentes, dans lequel les positions radiales d'un ensemble de pattes expansibles sont décalées par rapport à la position radiale d'un autre ensemble de pattes extensibles.

11. Filtre selon l'une quelconque des revendications précédentes, dans lequel le filtre est emboîté dans un cathéter dans un état non déployé.

12. Tube selon l'une quelconque des revendications 1 à 10, qui est le second tube.
